# EUROPEAN PATENT APPLICATION

(11) **EP 4 360 597 A1**
(43) Date of publication of application: **01.05.2024**
(21) Application number: 22827712.5
(22) Date of filing: 24.06.2022
(51) Int. Cl.: A61F 2/95, A61B 17/00

(54) **INTERVENTIONAL INSTRUMENT PREASSEMBLED DELIVERY SYSTEM, PREASSEMBLED DELIVERY ASSEMBLY, AND LOADING METHOD**

(30) Priority: 24.06.2021 CN 202110705318; 03.12.2021 CN 202111466057
(71) Applicant: Venus MedTech (HangZhou), Inc., Hangzhou, Zhejiang 310052 (CN)
(72) Inventor: GONG, Quangang, Hangzhou, Zhejiang 310052 (CN); CHEN, Mao, Hangzhou, Zhejiang 310052 (CN); FENG, Yuan, Hangzhou, Zhejiang 310052 (CN); LI, Yaru, Hangzhou, Zhejiang 310052 (CN); RUI, Dan, Hangzhou, Zhejiang 310052 (CN)
(74) Representative: Zenz Patentanwälte Partnerschaft mbB
(86) International application number: PCT/CN2022/101337
(87) International publication number: WO 2022/268220

(57) **Abstract**

A preloading and delivering system, a preloading and delivering assembly and a loading method for an interventional instrument. The interventional instrument preloading and delivering system includes a catheter component (260), a crimping device (240), an interventional instrument (90), a control handle (60) and a pulling wire (21). The catheter component (260) has opposing distal and proximal ends. The crimping device (240) has a cylindrical structure with an axis direction, in the axis direction, one end of the crimping device (240) surrounds a distal end of the catheter component (260), and the other end encloses a chamber with an inner conical surface. The interventional instrument (90) is radially deformable and has relative compressed and expanded states. The control handle (60) is connected to the proximal end of the catheter component (260). One end of the pulling wire (21) is controlled by the control handle (60), and the other end extends distally in the catheter component (260) and cooperates with the interventional instrument (90). The pulling wire (21) has a locked state and an unlocked state relative to the interventional instrument (90). In the locked state, the pulling wire (21) can pull the interventional instrument (90) to transform from the expanded state to the compressed state, and in the unlocked state, the pulling wire (21) can be disengaged from and release the interventional instrument (90). The interventional instrument (90), before being loaded, is in the expanded state, which facilitates the later deformation and release of the interventional instrument (90) in the human body.

## Description

### TECHNICAL FIELD

This disclosure relates to the field of medical device, and in particular to a preloading and delivering system, a preloading and delivering assembly and a loading method for an interventional instrument.

### DESCRIPTION OF THE PRIOR ART

With the development of medical technology, heart valve prostheses have been used to treat heart valve dysfunction. Native heart valves such as the aortic, pulmonary and mitral valves provide important functions in ensuring an adequate blood supply to the cardiovascular system. In some treatment cases, the native heart valves may deteriorate due to congenital, inflammatory or infectious reasons. This damage to the native heart valves may cause serious harm or even death.

In the related art, a common treatment method for native heart valve disease is to repair or replace the valve through surgery. In order to overcome the many complications easily caused by surgery, in related technologies, especially in transvascular technologies, flexible catheters are used for intervention and to implant the heart valve prosthesis. Transvascular techniques have less invasive procedures than surgical procedures such as open-heart surgery. In the transvascular techniques, the prosthetic valve in a loaded state is mounted on a distal portion of a flexible catheter and advanced through the patient's blood vessels until the prosthetic valve reaches the implantation site. The prosthetic valve at the end of the catheter expands to its functional size at the site of the diseased native valve.

During the implantation of a self-expandable prosthetic valve, when the operator begins to withdraw the delivery sheath to release the prosthetic valve, the prosthetic valve tends to "jump" out of the end of the sheath very quickly. In other words, the outward bias force of the stent of the prosthetic valve tends to cause the prosthetic valve to spring out of the distal end of the delivery sheath very quickly, making it difficult to deliver the prosthetic valve from the sheath in a precise and controlled manner and increasing the risk of trauma to the patient.

Secondly, in the existing valve products, usually before the valve is expanded to 2/3 of its length, the sheath is advanced to compress the valve stent and recover the valve; when the valve is fully expanded, because the end of the valve stent has been fully expanded, it is very difficult to recover the valve stent.

### SUMMARY OF THE DISCLOSURE

In order to solve the above technical problems, the present disclosure discloses an interventional instrument preloading and delivering system, including:
a catheter component having opposing distal and proximal ends;
a crimping device having a cylindrical structure with an axis direction, wherein in the axis direction, one end of the crimping device surrounds a distal end of the catheter component, and the other end encloses a chamber with an inner conical surface;
an interventional instrument radially deformable and having relative compressed and expanded states;
a control handle connected to the proximal end of the catheter component; and
a pulling wire, one end of which is controlled by the control handle, and the other end of which extends distally in the catheter component and cooperates with the interventional instrument, wherein the pulling wire has a locked state and an unlocked state relative to the interventional instrument, in the locked state, the pulling wire is configured to pull the interventional instrument to transform from the expanded state to the compressed state, and in the unlocked state, the pulling wire is configured to be disengaged from and release the interventional instrument.

Optionally, the catheter component includes:
an inner sheath, a distal end of which is provided with a locking portion;
an outer sheath slidably surrounding on an outside of the inner sheath, wherein the interventional instrument is configured to be loaded and received in the outer sheath in the compressed state; and
an inner core slidably arranged in the inner sheath, wherein one end of the inner core is an extension section extending out of the distal end of the inner sheath, a radial gap between the inner core and the inner sheath is a threading passage in which the pulling wire is movably arranged, and a lock member at a distal side of the locking portion is fixed on the extension section; the lock member has a locked position in which the lock member is engaged with the locking portion and an unlocked position in which the lock member is disengaged from the locking portion, and in the locked state, the pulling wire is wound around the interventional instrument and then around the lock member.

Optionally, a lock seat is fixed at the distal end of the inner sheath, and the locking portion is provided on the lock seat.

The extension section of the inner core is provided with a mounting seat, and a plurality of lock members are provided which are rod-shaped, each of which is connected to the mounting seat, and the lock members extend further proximally from the mounting seat.

The lock seat or the mounting seat is further provided with a guide hole, and the pulling wire extends outward from the guide hole.

Optionally, the locking portion is a locking hole or a locking groove.

Optionally, the catheter component further includes a wire control tube, the wire control tube is slidably fitted in the threading passage, and a proximal end of the pulling wire is fixedly connected to a distal end of the wire control tube and is controlled by the control handle through the wire control tube.

Optionally, relative to a distal port of the threading passage, when the lock member is in the locked position, the pulling wire has relative tightened and extended states.

When the pulling wire is in the tightened state, a joint between the interventional instrument and the pulling wire is close to the lock seat.

When the pulling wire is in the extended state, the interventional instrument is in the expanded state and located in the crimping device, and the joint between the interventional instrument and the pulling wire is relatively far away from the lock seat.

Optionally, the proximal end of the pulling wire and the distal end of the wire control tube is connected by the following arrangement:
the distal end of the wire control tube is fixed with a connecting ring, and the proximal end of the pulling wire is threaded through and fixed on the connecting ring.

Optionally, the lock member is surrounded by a slidable push block, a compression spring is arranged between the push block and the mounting seat, and an engagement portion between the lock member and the pulling wire is located at a proximal side of the push block.

Optionally, the push block is in a sheet shape and is provided with through holes for the lock members to pass through.

Optionally, a support sleeve is movably provided on the inner core, when the lock member is in the locked position, the support sleeve is in contact between the push block and the lock seat, (the compression spring deforms) and the lock member extends out of the push block to engage with the locking portion.

Optionally, a plurality of groups of pulling wires are provided, each group corresponds to a lock member and consists of a single pulling wire extending in one direction and having a wire loop at an end for movably surrounding the lock member.

Alternatively, each group consists of a single wire extending back and forth, with a wire loop formed at a turning point for movably surrounding the lock member.

Optionally, the lock seat includes a guide plate, a connecting sleeve and a fixed plate connected in sequence from a distal end to a proximal end, wherein the locking hole is opened in the fixed plate, the guide plate is provided with a guide hole corresponding to the locking hole.

The lock member passes through the guide hole in the locked position, and is inserted into the corresponding locking hole after passing over/across a periphery of the connecting sleeve, wherein a section of the lock member corresponding to the periphery of the connecting sleeve serves as a working section, and the pulling wire is constrained to the working section.

Optionally, the crimping device has a cylindrical structure with an axis direction, in the axis direction, the cylindrical structure includes:
a connection section having a length of 30 to 80 mm, one end of which is provided with a radially deformable locking mechanism;
a transition section enlarged relative to the connection section and having an inner conical surface for guiding the interventional instrument to radially deform, wherein the transition section is fixed to an end of the connection section away from the locking mechanism; and
an extension section, wherein the extension section and the transition section define a chamber for receiving the interventional instrument in the expanded state.

Optionally, the distal end of the outer sheath has a diameter-enlarged section for accommodating the interventional instrument, and the locking mechanism acts on the diameter-enlarged section.

The locking mechanism acts on the proximal end of the diameter-enlarged section.

Optionally, the locking mechanism includes:
a plurality of elastic claws located at an end of the connection section and distributed in a circumferential direction of the connection section at intervals; and
a sliding sleeve surrounding an outer periphery of the plurality of elastic claws and slidably matched with the connection section, wherein the sliding sleeve has relative fastening and release positions on its own sliding path, and wherein in the fastening position, the sliding sleeve holds and exerts force on the plurality of elastic claws inward, and in the release position, the sliding sleeve releases the force on the plurality of elastic claws.

Optionally, the locking mechanism includes:
an elastic ring located inside the connection section; and
a lock cap threadedly engaged with the connection section, wherein at least a part of the lock cap extends inside the connection section and abuts the elastic ring.

Optionally, in the axis direction, the chamber has a length greater than or equal to that of the interventional instrument in the expanded state.

Optionally, the transition section and the extension section are formed in separate pieces, and a junction of the two is located at a position of the chamber adjacent to the connection section.

Optionally, the action portion of the locking mechanism in the crimping device on the catheter component is located on the proximal side of the interventional instrument.

Optionally, the outer sheath, inner sheath, wire control tube and inner core respectively extend to the control handle.

The proximal end of the inner sheath is fixedly connected to the control handle.

The proximal ends of the outer sheath, the wire control tube and the inner core are movably connected to the control handle and are axially slidable relative to the control handle, and three driving mechanisms are provided in the control handle that are respectively in transmission fit with the outer sheath, the wire control tube and the inner core.

Optionally, the interventional instrument is a heart valve prosthesis and includes a stent and leaflets, the stent generally has a meshed cylindrical structure with a blood flow channel therein, and the leaflets cooperate with each other in the blood flow channel to open or close the blood flow channel; each leaflet includes a fixed edge fixed to the stent, and a free edge for cooperating with other leaflets to control the blood flow channel.

The stent includes:
an annular part, the annular part has a radially deformable structure, two ends of the annular part in its own axial direction are a first end and a second end respectively, wherein an edge of the first end includes a plurality of cell sections arranged in sequence in a circumferential direction of the annular part; and
a plurality of guide parts arranged at intervals in the circumferential direction of the annular part, one side of each guide part is connected to a corresponding cell section, and the other side of each guide part gradually tapers to an end, at which end a retrieval cell is provided for the pulling wire to pass through.

Optionally, the first end of the annular part is formed by connected ribs with peaks and valleys; each cell section includes at least 3 to 7 peaks.

The peaks are directly connected to the corresponding guide parts.

Optionally, in an outflow direction, the guide part includes a second region and a first region.

The first region is the retrieval cell for the pulling wire to pass through.

The second region includes a second cell for fixing ears of the leaflets.

Optionally, the second cell has a top end and a bottom end, the top end is connected to a bottom end of the retrieval cell, and the bottom end is connected to the peak of the corresponding cell section positioned centrally in the circumferential direction of the corresponding cell section.

Optionally, the guide parts are evenly arranged in the circumferential direction of the annular part; both the guide parts and the annular part have hollowed cell structures; in the axial direction of the annular part, the annular part includes a plurality of rows of cells; and an interface between the guide part and the annular part is formed by vertices of one circle of cells.

Optionally, depending on the blood flow direction controlled by the leaflets, the first end is an outflow end, and the second end is an inflow end, wherein two ends of the fixed edge are respectively located on two adjacent guide parts, and a middle portion of the fixed edge extends to the annular part.

Optionally, in the axial direction of the annular part, the interface between the guide part and the annular part is adjacent to the free edge of the leaflet.

Optionally, adjacent leaflets are connected to each other through joint portions fixed to the stent, and each joint portion is located on a corresponding guide part.

Optionally, the guide part includes four closed regions (each closed region can be understood as a cell. Different cells in this disclosure are not strictly limited to the same size and shape, but just for the overall structural characteristic description), each closed region corresponds to a cell, and the four closed regions are:
a first region being the retrieval cell;
a second region, in the circumferential direction of the annular part, the second region is aligned with the first region and serves as a central region of the guide part;
a third region located on one side of the central region in the circumferential direction of the annular part; and
a fourth region located on the other side of the central region in the circumference of the annular part.

Optionally, the sparse area further extends to the end of the guide part.

Optionally, a second cell is provided in the second region, and the second cell is used to fix the ears of the leaflets.

Optionally, the cell in the third and fourth regions adjacent to the first and second regions is a third cell, and the third cell has the largest area among all cells of the guide part.

Optionally, in a circle of cells at the second end of the annular part, each cell is quadrangular, and the two sides near the second end are longer than the two sides near the first end.

Optionally, an inner side of the stent is provided with an inner covering film, and the inner covering film is located on an inflow end of the leaflets and is fixed with the fixed edges of the leaflets.

A perivalvular leakage preventing component is fixed on an outside of the inner covering film, and the perivalvular leakage preventing component consists of spaced pieces corresponding to the cells.

Optionally, the perivalvular leakage preventing component and the inner covering film are formed in one piece, and in the expanded state, the perivalvular leakage preventing component extends outward in a radial direction of the stent from the corresponding cells.

Optionally, the inner covering film is made of PET material, and the perivalvular leakage preventing component is made of porous material.

Optionally, the perivalvular leakage preventing component is located on an inflow end of the stent. In the same piece of perivalvular leakage preventing component, the highest protruding portion is closer to an inflow end of the corresponding cell.

Optionally, a distance between the highest protruding portion of the same piece of perivalvular leakage preventing component and the inflow end of the corresponding cell is S1, and a distance between the highest protruding portion of the same piece of perivalvular leakage preventing component and an outflow end of the corresponding cell is S2, where S1 : S2 is 0 to 0.8. (0.3 to 0.8 is preferred.)

Optionally, the same piece of perivalvular leakage preventing component gradually becomes thicker from the outflow end toward the inflow end, and then gradually becomes thinner after reaching the highest protruding portion.

This disclosure further provides an interventional instrument preloading and delivering system, including:
a catheter component having opposing distal and proximal ends;
a crimping device having a cylindrical structure with an axis direction, wherein in the axis direction, one end of the crimping device surrounds a distal end of the catheter component, and the other end encloses a chamber with an inner conical surface;
an interventional instrument including a stent with a cylindrical structure and having relative compressed and expanded states, the interventional instrument is configured to be located in the chamber in the expanded state, the stent including:
   an annular part, the annular part has a radially deformable structure, two ends of the annular part in its own axial direction are a first end and a second end respectively, wherein an edge of the first end includes a plurality of cell sections arranged in sequence in a circumferential direction of the annular part; and
   a plurality of guide parts arranged at intervals in the circumferential direction of the annular part, one side of each guide part is connected to a corresponding cell section, and the other side of each guide part gradually tapers to an end, at which end a retrieval cell is provided;
a control handle connected to the proximal end of the catheter component; and
a pulling wire, one end of which is controlled by the control handle and the other end extends distally in the catheter component and engages with the retrieval cell of the interventional instrument; relative to the interventional instrument, the pulling wire has a locked state and an unlocked state, in the locked state, the pulling wire is configured to pull the interventional instrument to transform from the expanded state to the compressed state, and in the unlocked state, the pulling wire is configured to be disengaged from and release the interventional instrument.

This disclosure further discloses an interventional instrument preloading and delivering assembly, including:
a packaging component at least including a carrying tray, the carrying tray including a tray body, the tray body having opposing top and bottom sides, and the top side of the tray body having a recessed portion that forms an accommodation area; and
an interventional instrument preloading and delivering system, the interventional instrument preloading and delivering system is the above-mentioned interventional instrument preloading and delivering systems and is located in the accommodation area.

Optionally, the accommodation area includes:
a handle accommodation area, the control handle is located in the handle accommodation area;
an interventional instrument accommodation area, the crimping device and the interventional instrument are located in the interventional instrument accommodation area; and
a catheter component accommodation area, at least a part of the catheter component is located in the catheter component accommodation area.

This disclosure further discloses a loading method for an interventional instrument, including:
maintaining the interventional instrument in an expanded state and connecting it to a control handle through a pulling wire;
providing a crimping device with an inner conical surface around an outer periphery of the interventional instrument, pulling the pulling wire, and gradually compressing the interventional instrument radially under guidance of the inner conical surface; and
receiving the radially compressed portion of the interventional instrument into an outer sheath until the interventional instrument completely enters the outer sheath.

Specific benefits will be further explained in combination with specific structures or steps in the description of embodiments.

### BRIEF DESCRIPTION OF DRAWINGS

FIG. 1a is a schematic structural view of an interventional instrument preloading and delivering system according to an embodiment of the present disclosure;
FIG. 1b is a schematic view showing the resistance point during the retrieval of the stent in the prior art;
FIG. 2a is a schematic view of a stent according to an embodiment;
FIG. 2b is a schematic top view of the stent in FIG. 2a;
FIG. 2c is a schematic view showing the axial length proportional relationship of the parts of the stent in FIG. 2a;
FIGS. 2d and 2e are schematic views of the stent in FIG. 2a from different perspectives;
FIG. 2f is an enlarged view of the guide part of the stent in FIG. 2a;
FIG. 2g is an enlarged view of a retrieval cell of the stent in FIG. 2a;
FIG. 2h is a perspective view of a stent according to another embodiment;
FIG. 2i is a front view of the stent of FIG. 2h;
FIG. 2j is an enlarged view of the guide part in FIG. 2h;
FIG. 2k is a perspective view of a stent according to another embodiment;
FIG. 21 is a front view of the stent in FIG. 2k;

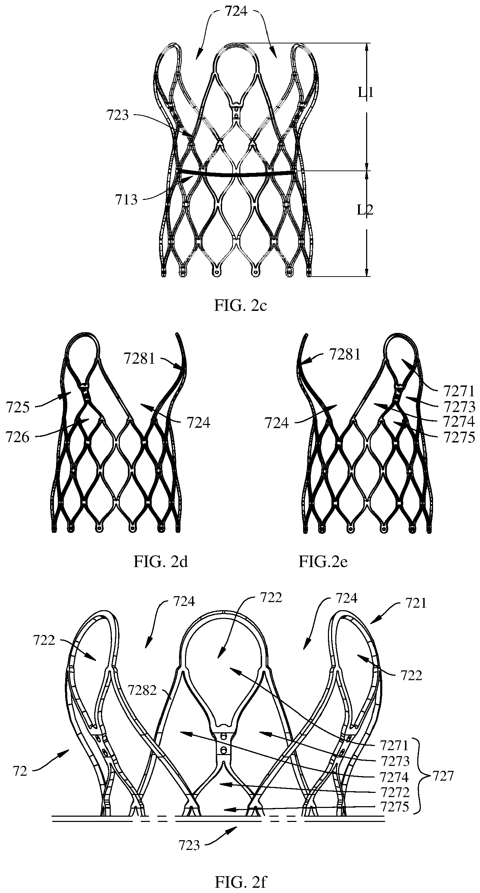

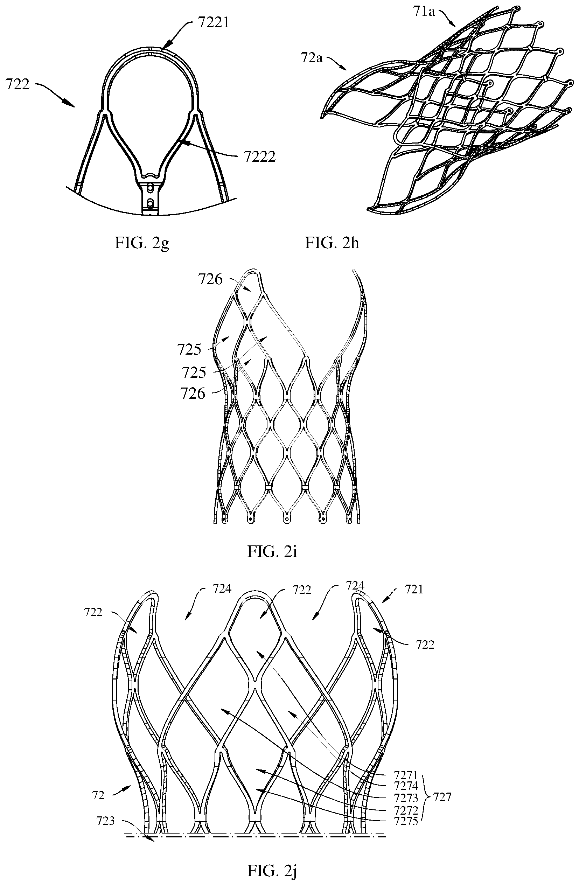

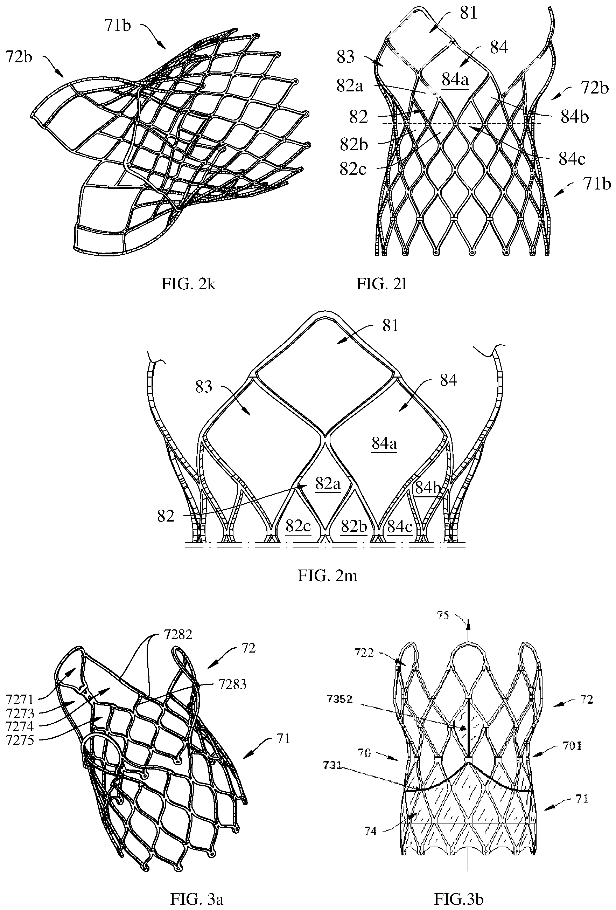

FIG. 2m is an enlarged view of the guide part in FIG. 2k;
FIG. 3a is a schematic view of a stent according to an embodiment;

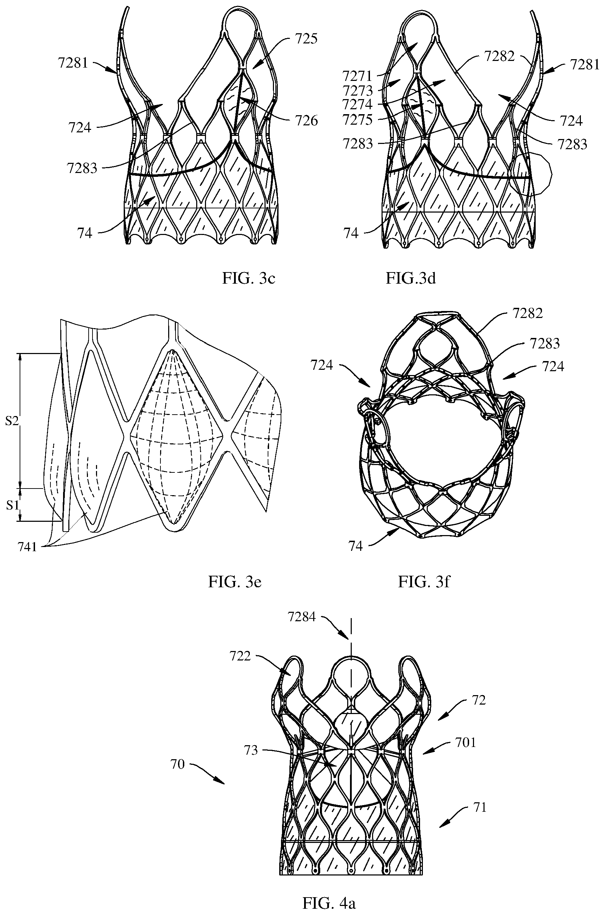

FIGS. 3b, 3c and 3d are schematic views of the stent in FIG. 3a from different perspectives;
FIG. 3e is an enlarged view of the circled portion in FIG. 3a;
FIG. 3f is a perspective schematic view of the stent in FIG. 3a from the top perspective;
FIG. 4a is a schematic view of a heart valve prosthesis device according to an embodiment;

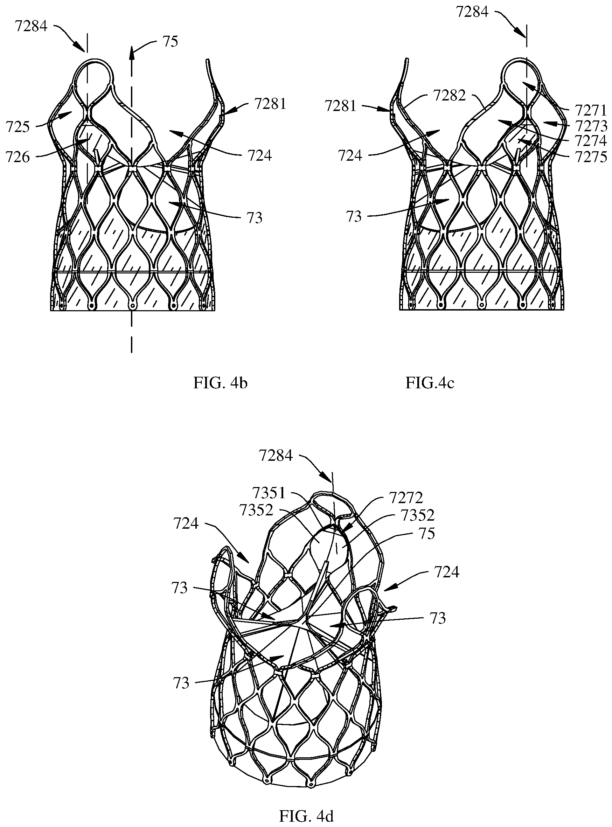

FIGS. 4b, 4c and 4d are schematic views of the heart valve prosthesis device in FIG. 4a from different perspectives;

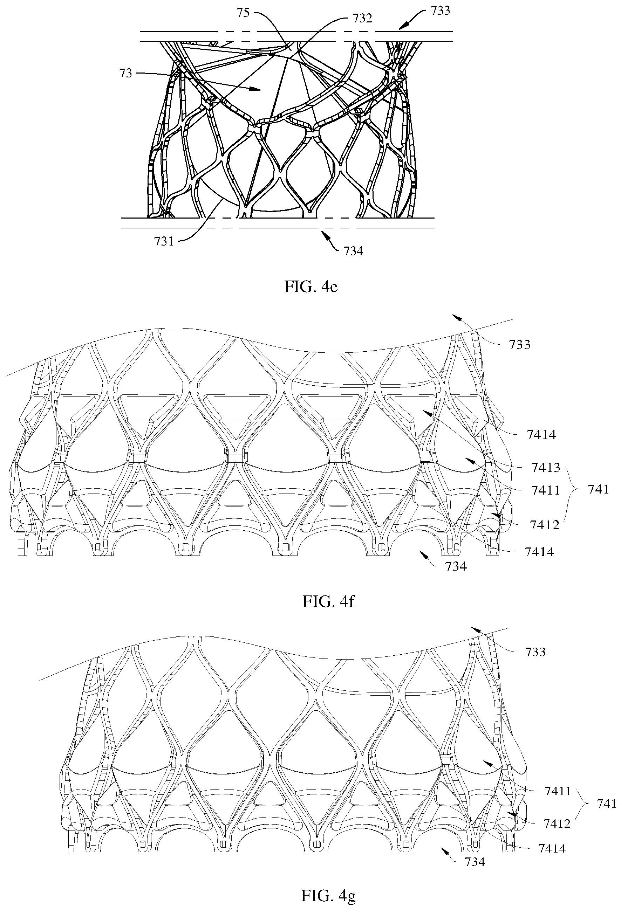

FIG. 4e is an enlarged view of the heart valve prosthesis device in FIG. 4d;
FIG. 4f is a schematic view of a perivalvular leakage preventing component according to another embodiment;
FIG. 4g is a schematic view of a perivalvular leakage preventing component according to another embodiment;

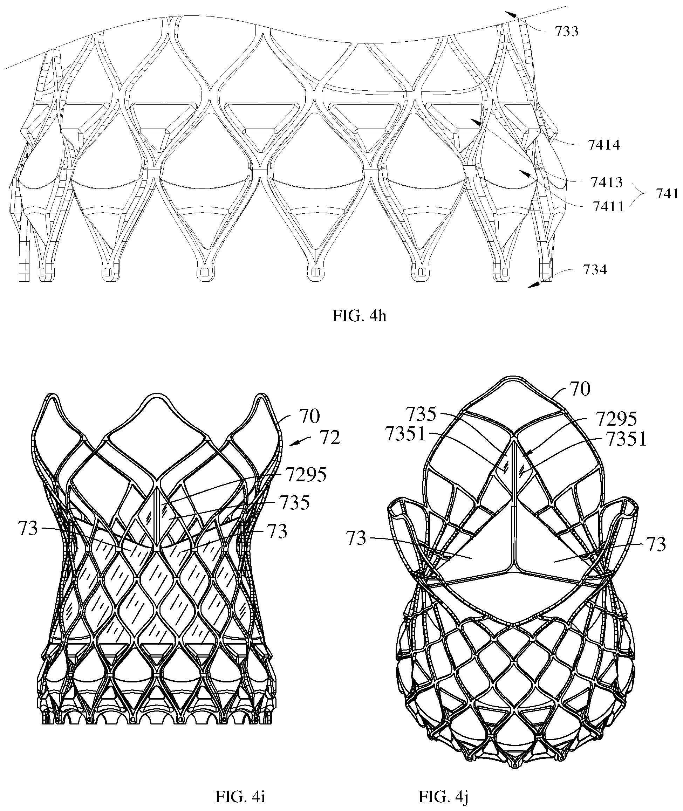

FIG. 4h is a schematic view of a perivalvular leakage preventing component according to another embodiment;
FIG. 4i is a schematic structural view showing the joint portion of the heart valve prosthesis device according to an embodiment;
FIG. 4j is a perspective view of FIG. 4i;

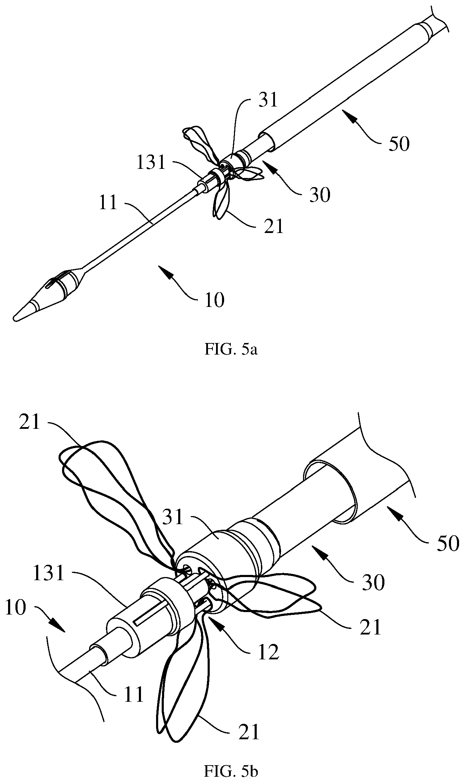

FIG. 5a is a schematic view of the distal side of the delivering system according to an embodiment of the present disclosure;
FIG. 5b is an enlarged view of the delivering system in FIG. 5a;

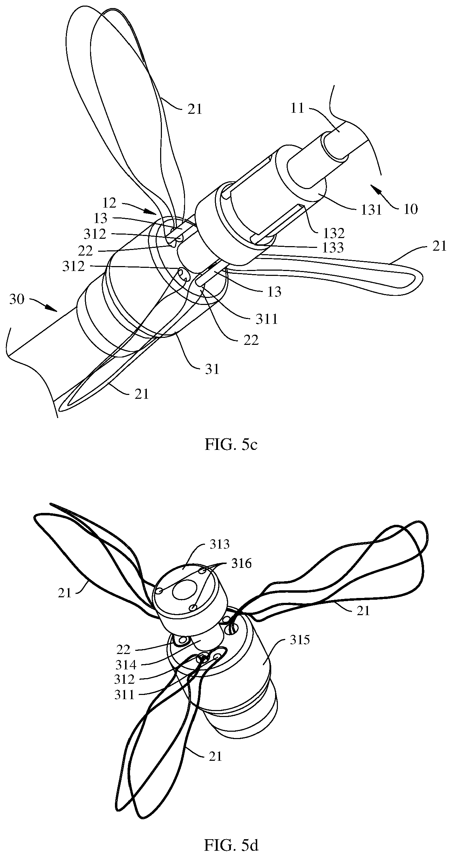

FIG. 5c is an enlarged view of the delivering system in FIG. 5a from another perspective;
FIG. 5d is an enlarged view of the lock seat and the pulling wire in the delivering system in FIG. 5a;

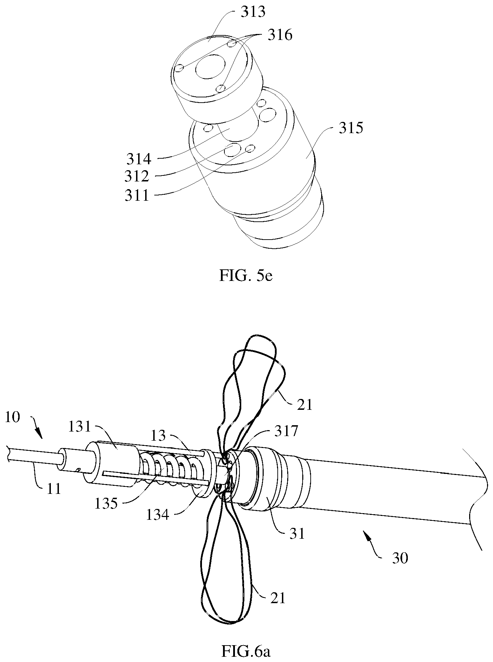

FIG. 5e is an enlarged view of the lock seat in the delivering system in FIG. 5a;
FIG. 6a is a schematic view of the distal side of the delivering system according to another embodiment of the present disclosure;

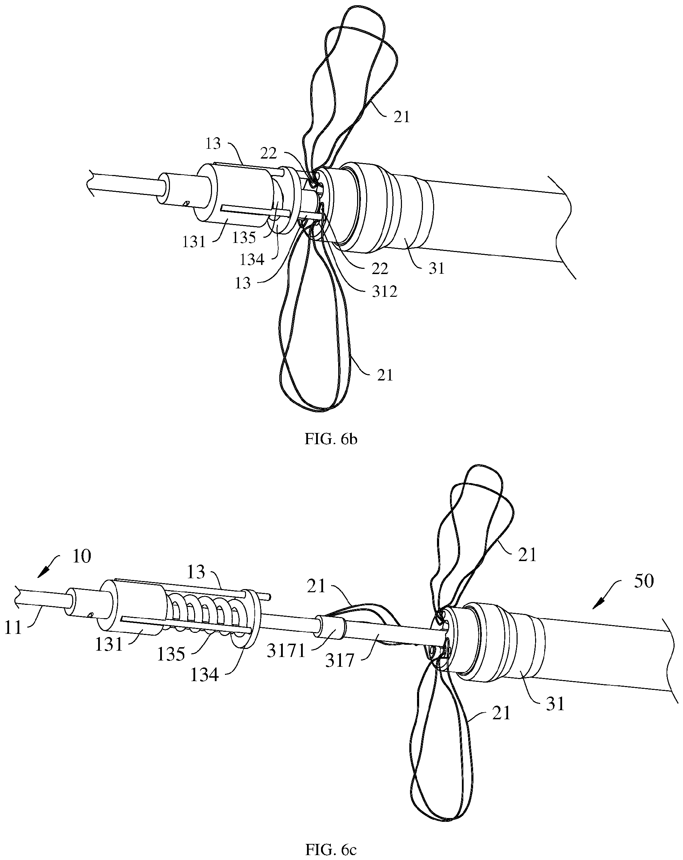

FIG. 6b is a schematic view of the lock member and the lock seat in FIG. 6a with the lock member inserted in the lock seat;
FIG. 6c is an exploded view of FIG. 6a;

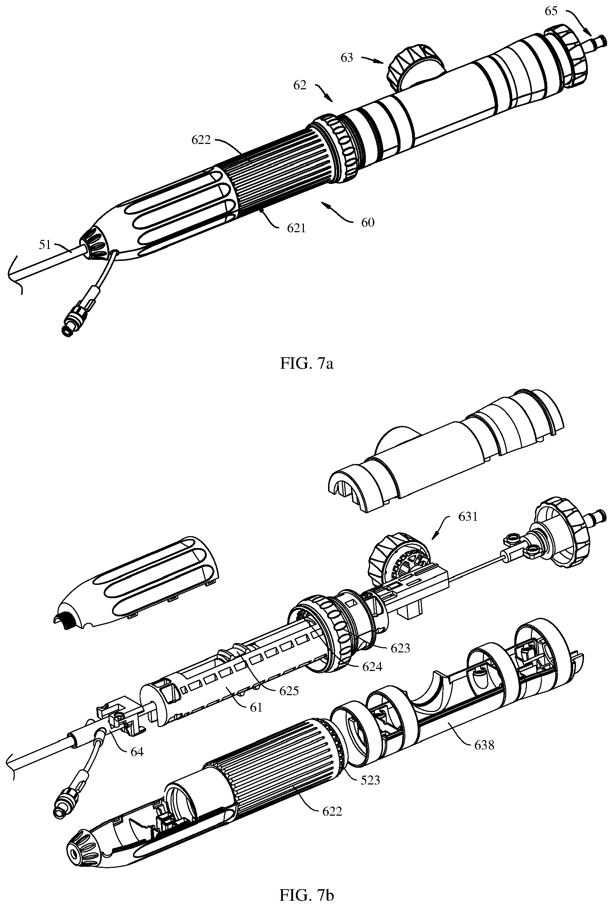

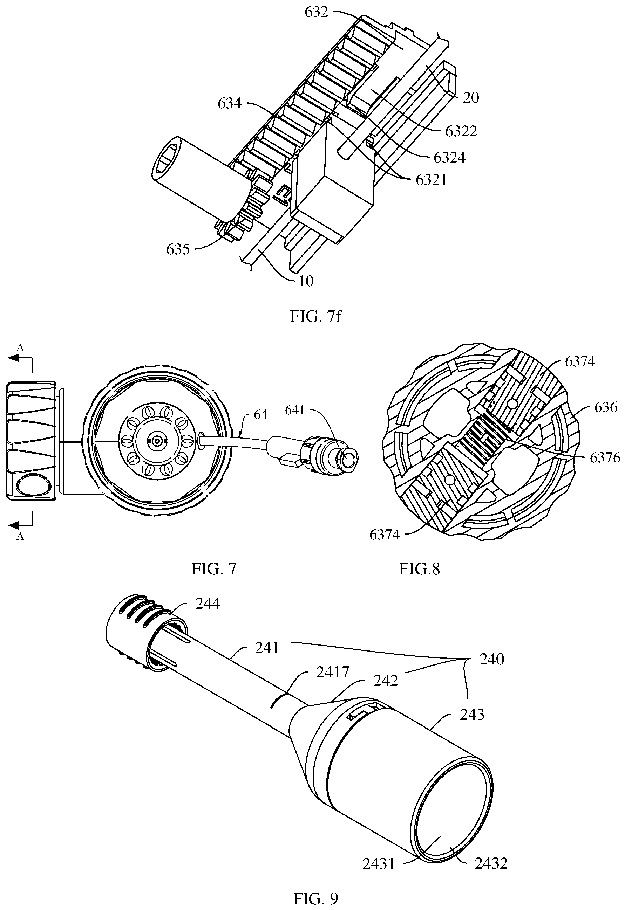

FIG. 7a is a perspective view of a control handle according to another implementation;
FIG. 7b is an exploded view of the control handle in FIG. 7a;

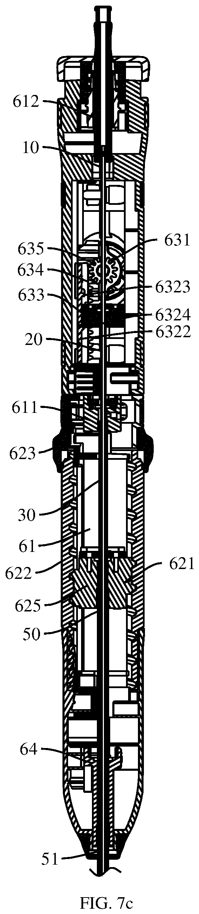

FIG. 7c is a cross-sectional view of the control handle in FIG. 7a;

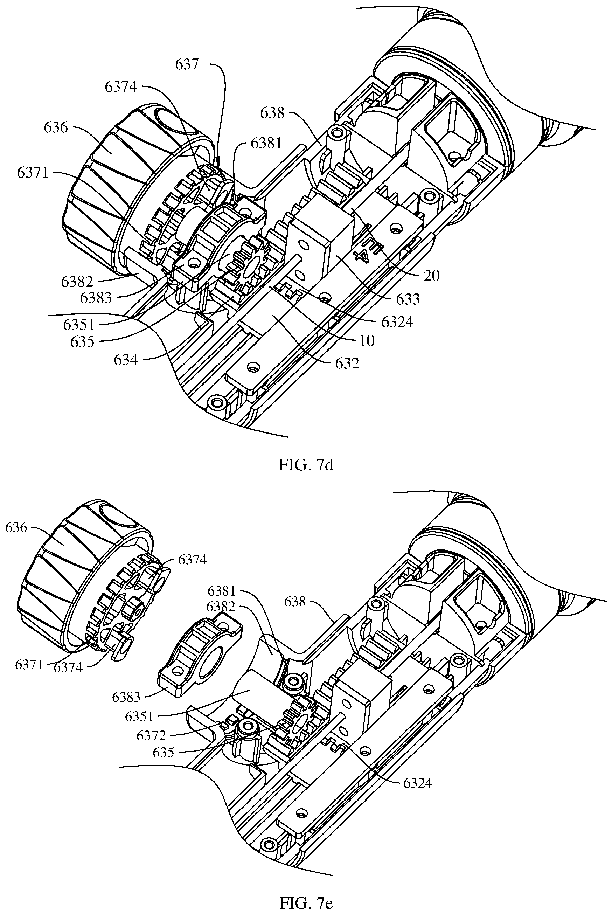

FIG. 7d is a partial structural view of the control handle in FIG. 7b showing the rack and pinion;
FIG. 7e is an exploded view of FIG. 7d;
FIG. 7f is a schematic structural view of the pinion, rack and clamping seat in FIG. 7d;
FIG. 7 is a front view of the control handle in FIG. 7a;
FIG. 8 is a cross-sectional view of portion A-A in FIG. 7h;
FIG. 9 is a perspective view of a crimping device according to an embodiment of the present disclosure;

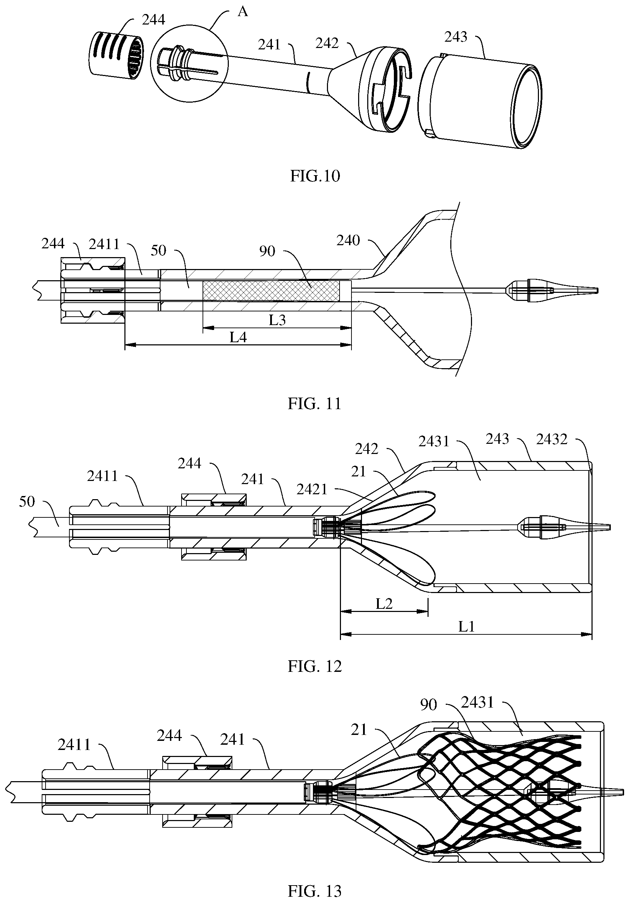

FIG. 10 is an exploded view of a crimping device according to an embodiment of the present disclosure;
FIG. 11 is a structural view showing the valve in a loaded state according to an embodiment of the present disclosure;
FIG. 12 is a structural view showing the crimping device and the outer sheath assembled together according to an embodiment of the present disclosure;
FIG. 13 is a structural view showing the connection between the pulling wire and the interventional instrument according to an embodiment of the present disclosure;

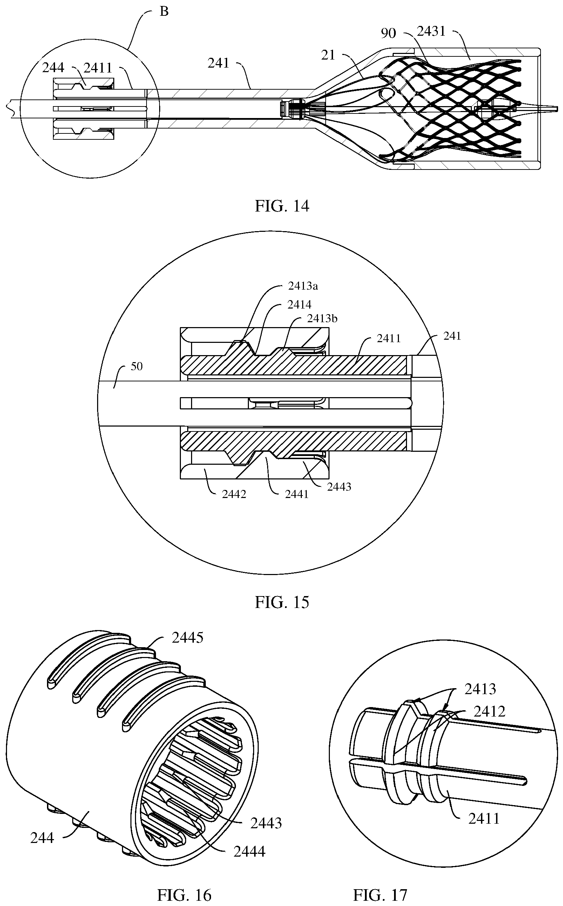

FIG. 14 is a structural view showing the locking mechanism in the fastening position according to an embodiment of the present disclosure;
FIG. 15 is an enlarged view of portion B in FIG. 14;
FIG. 16 is an enlarged view of a sliding sleeve of the locking mechanism according to an embodiment of the present disclosure;
FIG. 17 is an enlarged view of part A in FIG. 10;

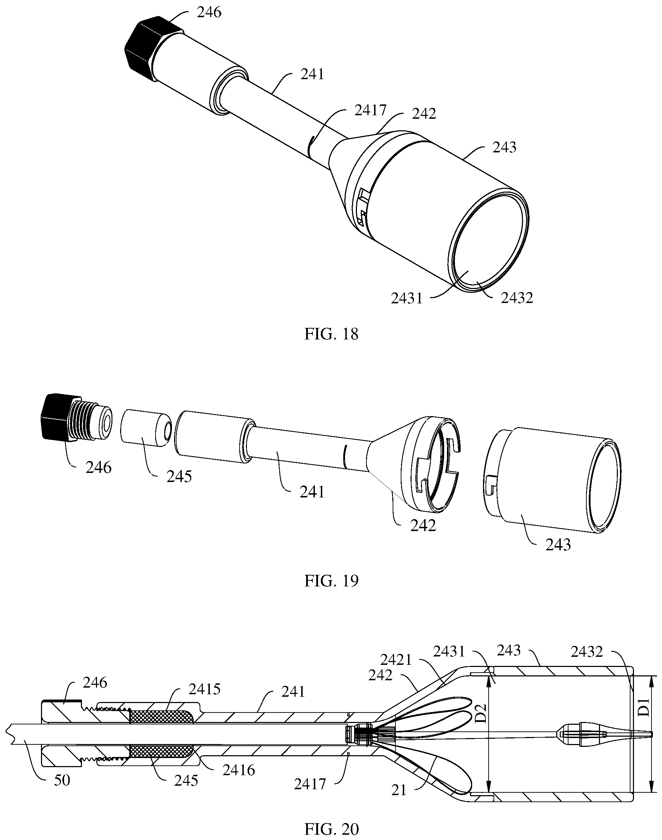

FIG. 18 is a structural view of a crimping device according to another embodiment of the present disclosure;
FIG. 19 is an exploded view of a crimping device according to another embodiment of the present disclosure;
FIG. 20 is a structural view showing a crimping device and an outer sheath assembled together according to another embodiment of the present disclosure;

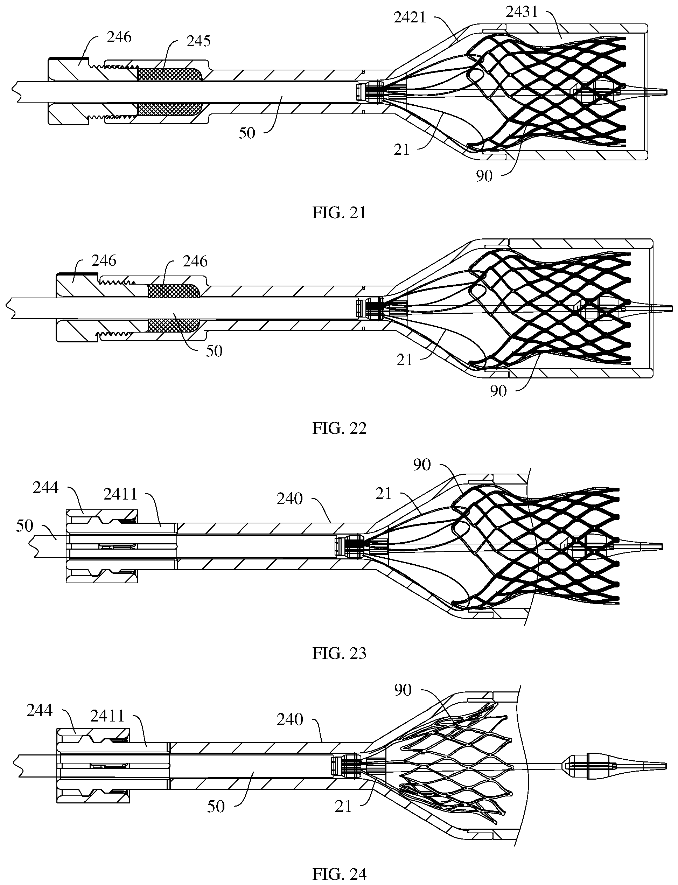

FIG. 21 is a structural view showing the connection between the pulling wire and the interventional instrument according to another embodiment of the present disclosure;
FIG. 22 is a structural view showing the locking mechanism in the fastening position according to another embodiment of the present disclosure;
FIG. 23 is a structural view showing an interventional instrument ready for being loaded according to an embodiment of the present disclosure;
FIG. 24 is a structural view showing the interventional instrument being compressed by the transition section according to an embodiment of the present disclosure;

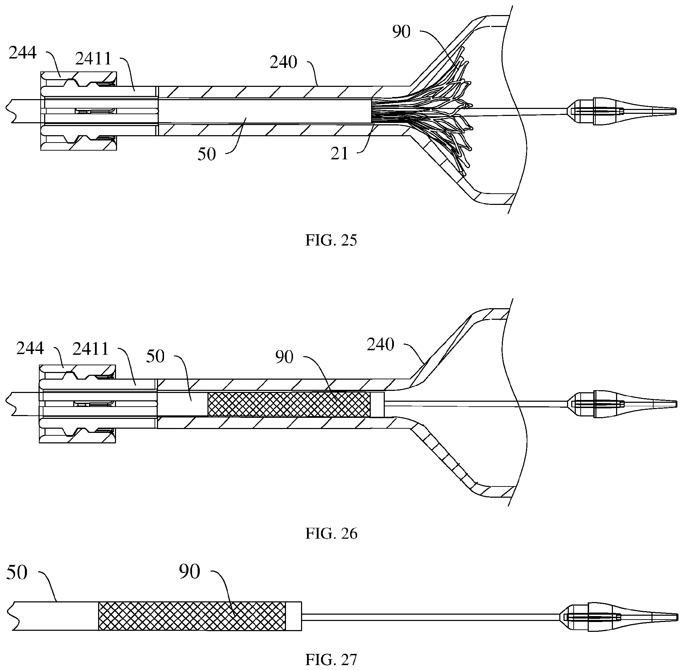

FIG. 25 is a structural view showing the crimping device and the outer sheath sliding distally to receive the interventional instrument according to an embodiment of the present disclosure;
FIG. 26 is a structural view showing the interventional instrument being loaded according to an embodiment of the present disclosure;
FIG. 27 is a structural view showing the crimping device separated from the outer sheath according to an embodiment of the present disclosure;

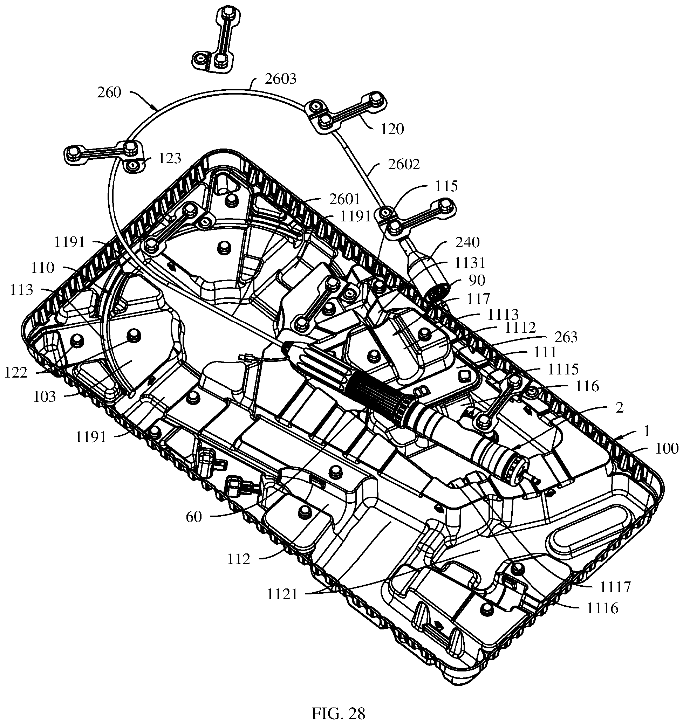

FIG. 28 is an exploded view of a delivering system and a tray according to an embodiment of the present disclosure;

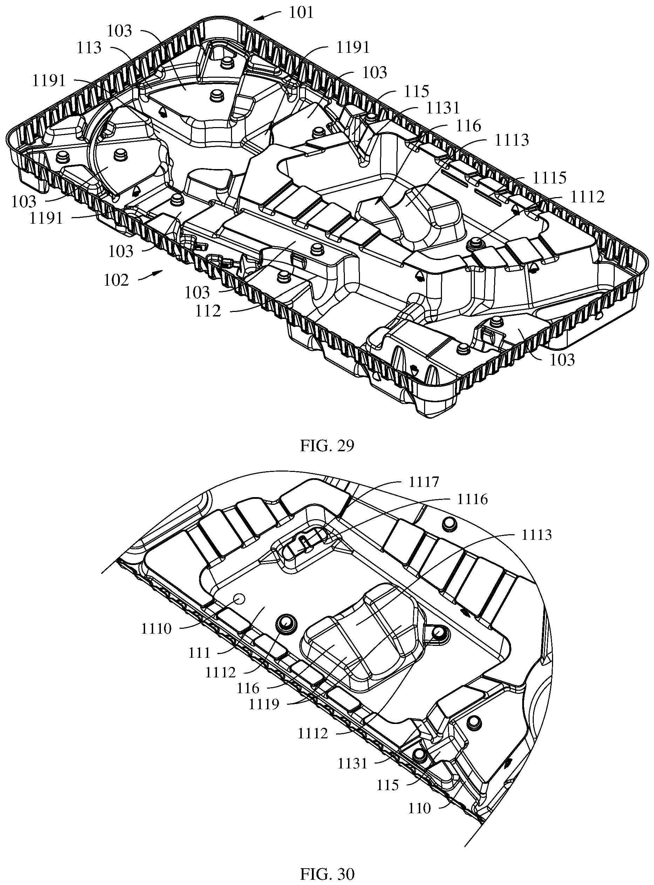

FIG. 29 is a perspective view of the tray according to an embodiment of the present disclosure;
FIG. 30 is a partial perspective view of the liquid tank of the tray from another perspective according to an embodiment of the present disclosure;

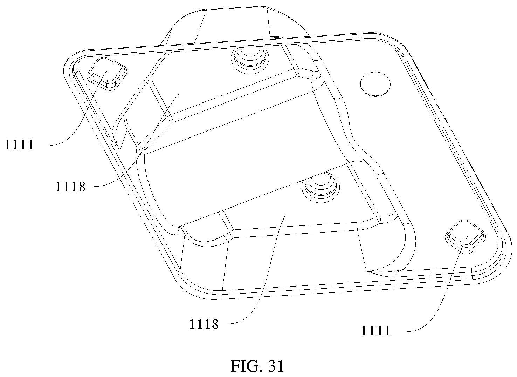

FIG. 31 is a perspective view of the extension mount viewed from the bottom side toward the top side according to an embodiment of the present disclosure;

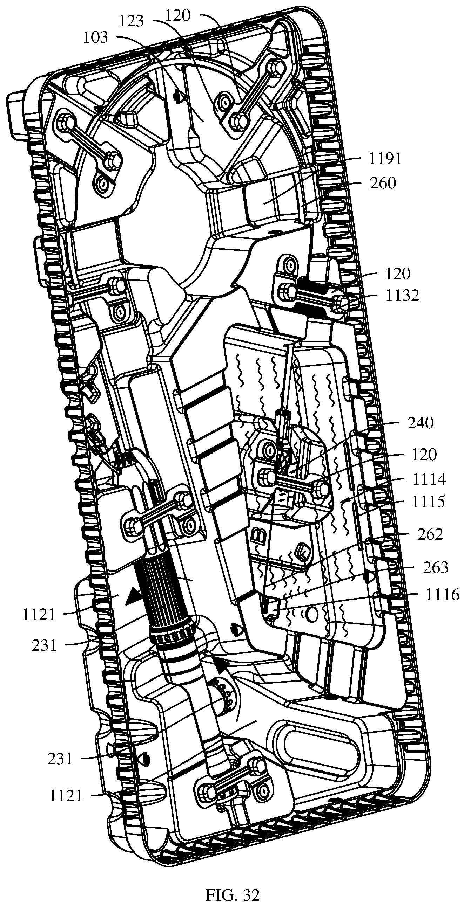

FIG. 32 is a perspective view of the implant delivering system loaded on a tray according to an embodiment of the present disclosure;

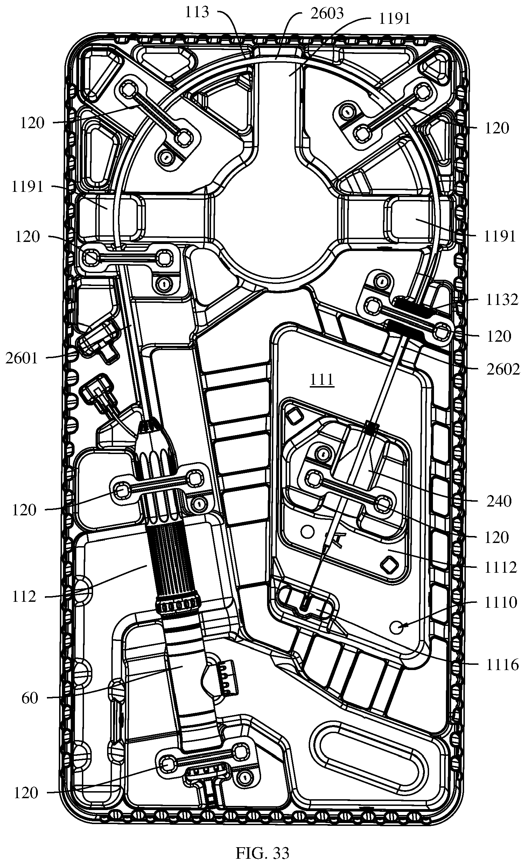

FIG. 33 is a front view of the implant delivering system loaded on a tray according to an embodiment of the present disclosure;

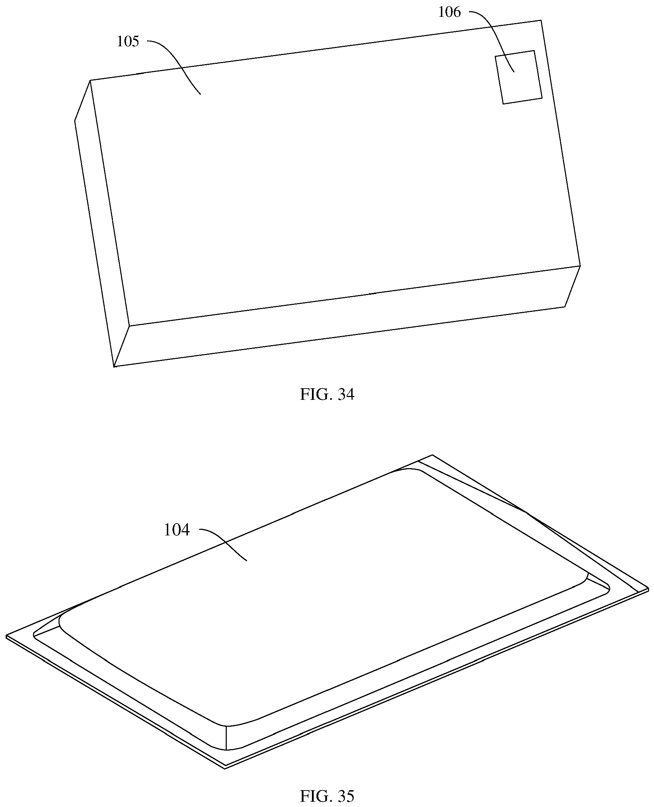

FIG. 34 is a perspective view of a packaging box of a packaging component according to an embodiment of the present disclosure;
FIG. 35 is a perspective view of a sealing bag of a packaging component according to an embodiment of the present disclosure;

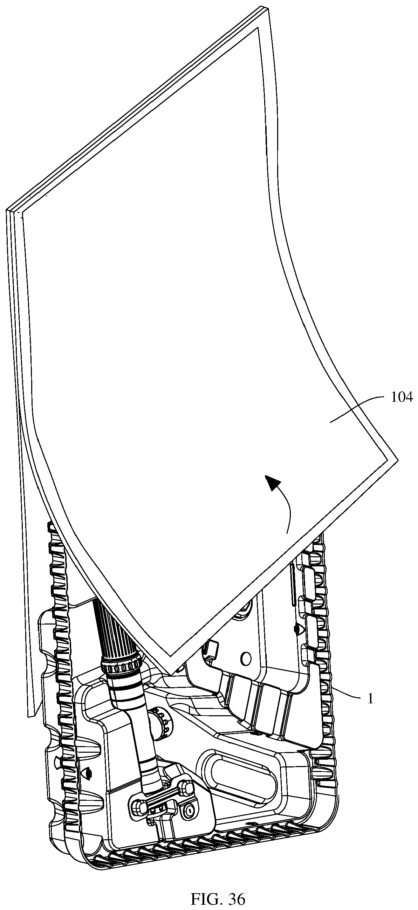

FIG. 36 is a perspective view showing a tray removed from the sealing bag according to an embodiment of the present disclosure;

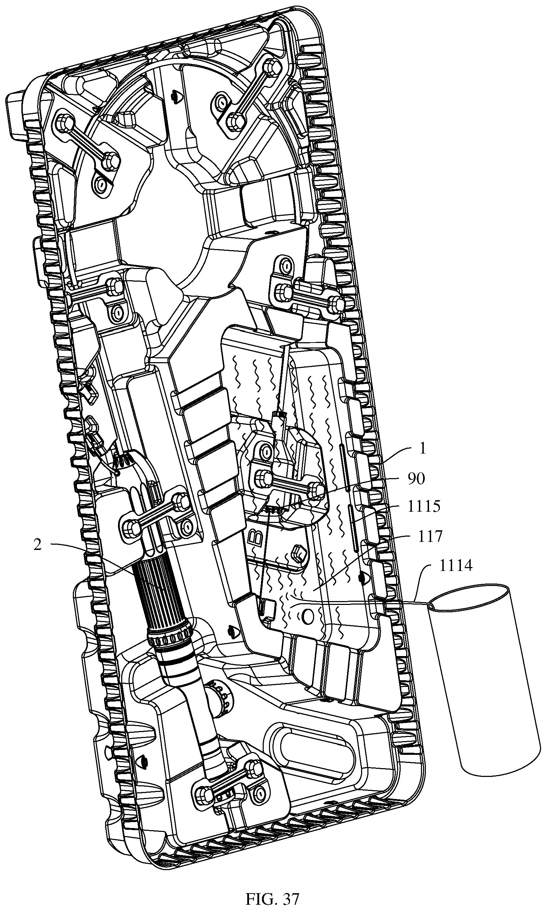

FIG. 37 is a perspective view showing pouring ice water into the liquid tank;

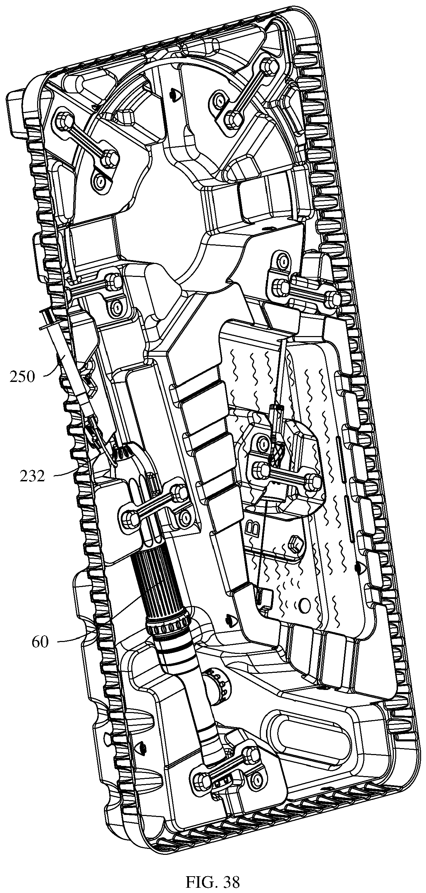

FIG. 38 is a perspective view showing the exhaust device being connected to the extension tube for exhaust operation; and

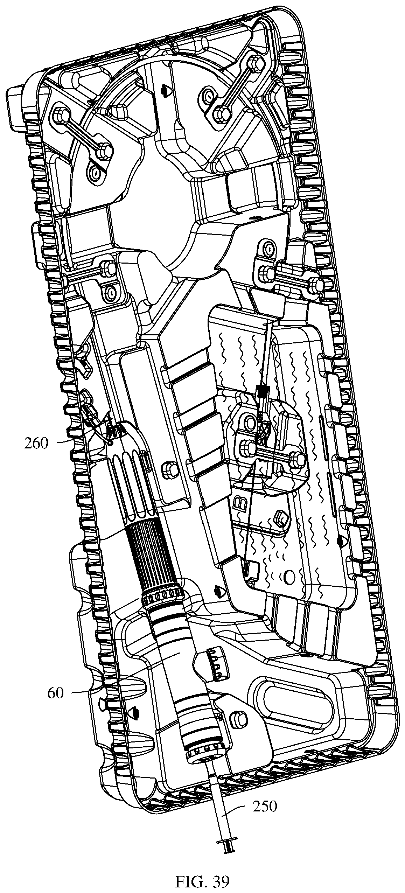

FIG. 39 is a perspective view showing the exhaust device being connected with the proximal end of the control handle for exhaust operation.

### List of reference symbols

1, tray; 100, tray body; 101, top side; 102, bottom side; 103, installation surface; 104, sealing bag; 105, packing box; 106, temperature display; 110, accommodation area; 111, liquid tank; 1110, liquid drain port; 1111, snap-in groove; 1112, clamping block; 1113, positioning groove; 1114, ice water; 1115, mark indication; 1116, positioning step; 1117, limiting groove; 1118, limiting groove; 1119, limiting protrusion; 112, handle accommodation area; 1121, avoidance area; 113, catheter component accommodation area; 1131, guide portion; 1132, sealing plug; 114, interventional instrument accommodation area; 115, sealing plug accommodation area; 116, fixed mount; 117, extension mount; 118, plug; 1191, avoidance area; 120, limiting member; 122, snapping post; 123, handle portion;
2, delivering system; 260, catheter component; 261, guide head; 262, steel wire; 263, positioning head; 2601, first section; 2602, second section; 2603, third section; 231, operating portion; 232, extension tube; transition section; 250, exhaust device; 240, crimping device; 241, connection section; 2411, elastic claw; 2412, rib; 2413, protruding ring; 2413a, first protruding ring; 2413b, second protruding ring; 2414, groove; 2415, installation chamber; 2416, step structure; 2417, assembly indicator; 242, transition section; 2421, inner conical surface; 243, extension section; 2431, chamber; 2432, opening; 244, sliding sleeve; 2441, protrusion; 2442, first region; 2443, second region; 2444, friction-reducing rib; 2445, anti-slip rib; 245, elastic ring; 246, lock cap;
10, inner core; 11, extension section; 12, threading passage; 13, lock member; 131, mounting seat; 132, mounting hole; 134, push block; 135, compression spring; 20, wire control tube; 21, pulling wire; 211, driving end; 212, working end; 22, engagement loop; 23, connecting ring; 30, inner sheath; 31, lock seat; 311, locking hole; 312, guide hole; 313, guide plate; 314, connecting sleeve; 315, fixed plate; 316, guide hole; 317, support sleeve; 40, bending piece; 50, outer sheath; 60, control handle; 61, support body; 611, fixed seat; 612, sliding seat; 62, thread fitting area; 621, thread connection component; 622, driving ring; 623, positioning ring; 624, positioning tooth; 625, clamping member; 63, rack-and-pinion fitting area; 631, pinion connection component; 632, base; 6321, slide rail; 6322, resilient snap; 6323, first limiting protrusion; 6324, second limiting protrusion; 633, clamping seat; 634, rack; 6341, first limiting protrusion; 6342, second limiting protrusion; 635, pinion; 6351, rotation shaft; 636, driving portion; 637, connection locking mechanism; 6371, first locking tooth; 6372, second locking tooth; 6373, maintaining component; 6374, snapping post; 6375, snap-in groove; 6376, reset member; 6377, locking pin; 6378, hole; 64, exhaust component; 65, positioning mechanism; 638, support cylinder; 6381, snapping block; 6382, outer protrusion; 6383, limiting seat; 70, stent; 701, waist; 702, resistance point; 71, annular part; 711, first end; 712, second end; 713, cell section; 72, guide part; 721, end; 722, retrieval cell; 7221, pulling section; 7222, transition section; 723, connection side; 724, opening; 725, sparse area; 726, dense area; 727, closed region; 7271, first region; 7272, second region; 7273, third region; 7274, fourth region; 7275, central region; 7281, maximum outer diameter; 7282, edge strut; 7283, branch strut; 7284, axis of symmetry; 73, leaflets; 731, fixed edge; 732, free edge; 733, outflow end; 734, inflow end; 735, joint portion; 7351, second cell; 7352, ear; 74, inner covering film; 741, perivalvular leakage preventing component; 7411, first circle of perivalvular leakage preventing component; 7412, second circle of perivalvular leakage preventing component; 7413, third circle of perivalvular leakage preventing component; 75, blood flow channel; 90, interventional instrument; 91, connector; 92, connecting ear.

### DESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this disclosure.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes one or more of the listed options in any combinations, or the combination of all of the listed options.

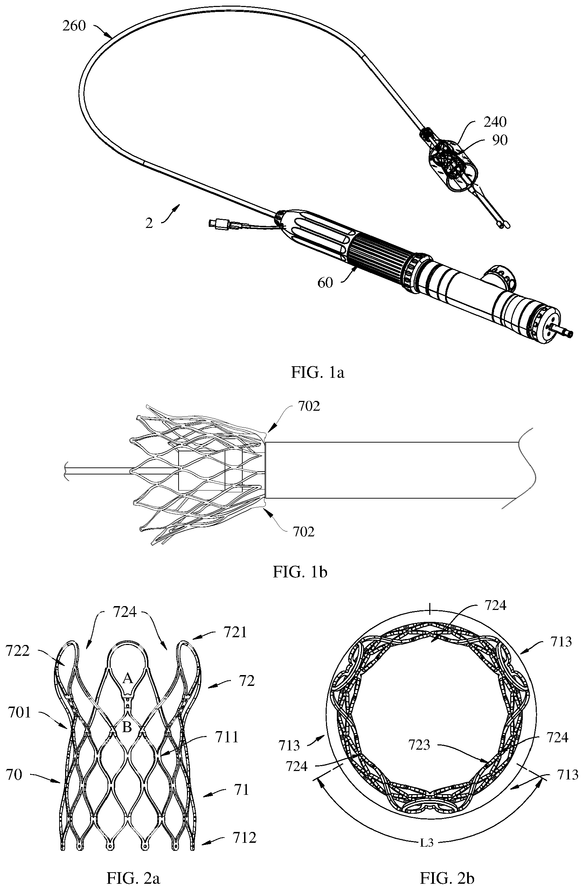

Referring to FIG. 1a, an interventional instrument preloading and delivering system is disclosed, which can be used to load an interventional instrument before surgery, and includes:
a catheter component 260 having opposing distal and proximal ends;
a crimping device 240 having a cylindrical structure with an axis direction, in the axis direction, one end of the crimping device 240 surrounds the distal end of the catheter component 260, and the other end defines a chamber 2431 having an inner conical surface 2421;
an interventional instrument 90, which is radially deformable and has relative compressed and expanded states;
a control handle 60 connected to the proximal end of the catheter component 260; and
a pulling wire 21, one end of which is controlled by the control handle 60 and the other end extends distally in the catheter component and engages with the interventional instrument 90. Relative to the interventional instrument 90, the pulling wire 21 has a locked state, in which the pulling wire 21 can pull the interventional instrument to transform from the expanded state to the compressed state, and an unlocked state, in which the pulling wire 21 is disengaged from and releases the interventional instrument.

The interventional instrument is in the expanded state before loading, which is conducive to the later deformation and release of the interventional instrument in the human body. During the loading process, through the cooperation of the crimping device and the pulling wire, the interventional instrument is guided to transform to the compressed state and stored in the catheter component 260 so as to complete the loading. Further, the pulling wire can be used to release and retrieve the interventional instrument in the human body. The interventional instrument is radially deformable and has relative compressed and expanded states. In this embodiment, a heart valve prosthesis (valve, for short) is taken as an example. The interventional instrument, without considering the stress in vivo, is fully expanded in the expanded state, which is equivalent to a pre-shaped profile after heat treatment. The process from the expanded state to the compressed state can be regarded as an intermediate state, in which the interventional instrument is at least partially compressed. The heart valve prosthesis includes a stent and leaflets. The stent generally has a meshed and cylindrical structure, with a blood flow channel therein. There are a plurality of leaflets which cooperate with each other in the blood flow channel to open or close the blood flow channel. Referring to FIG. 1b, when releasing the existing stent, due to the self-expanding characteristic of the stent, resistance points 702 usually occur to interfere with the delivering system, affecting the release process (especially the retrieval process) of the stent. Therefore, an improved inventive stent for the interventional instrument preloading and delivering system is disclosed. Referring to FIGS. 2a to 4e, the stent 70 according to an embodiment of the present disclosure includes:
an annular part 71, which has a radially deformable structure, and the two ends thereof in its own axial direction are a first end 711 and a second end 712 respectively. The edge of the first end 711 includes a plurality of cell sections 713 arranged in sequence in the circumferential direction of the annular part 71; and
a plurality of guide parts 72, which are arranged at intervals in the circumferential direction of the annular part 71. One side of each guide part 72 is connected to a corresponding cell section 713, and the other side gradually tapers to the end 721, where a retrieval cell 722 is provided.

Through the guide part 72, especially the retrieval cell 722, the stent 70 can be controlled throughout the whole process. Further, the arrangement and shape of the guide part 72 can reduce the impact of the self-expanding characteristic of the stent 70 on its retrieval, improving the operating experience of the medical staff and the therapeutic effect. With respect to the proportion of the guide part 72, referring to the embodiment shown in FIGS. 2b to 2c, the stent 70 has relative compressed and expanded states due to the radial deformation characteristic. In the expanded state, the axial direction of the annular part 71 is the length direction, the length of the guide part 72 is L1, the length of the annular part 71 is L2, and L1: L2 = 1: 0.5 to 1.5. In a specific product, L1: L2 = 1: 0.6 to 1.2. From another perspective, in the circumferential direction of the annular part 71, the length of the connection side 723 is L3 and L1: L3 = 0.5 to 1.5. The length L3 of the connection side 723 corresponds to the arc length of the circumferential surface of the annular part 71. In this regard, the stents 70 shown in FIGS. 3a to 3f and FIGS. 4a to 4e are similar to the stent 70 shown in FIGS. 2b to 2c and will not be repeated again.

With respect to the connection of the guide part 72, referring to the embodiment shown in FIG. 2f, in the axial direction of the annular part 71, one side of each guide part 72 is the connection side 723 that is connected with the corresponding cell section 713, and the connection sides 723 of two adjacent guide parts 72 are connected to each other. Alternatively, the connection sides 723 of two adjacent guide parts 72 can be spaced from each other. In any case, all the guide parts 72 are evenly arranged in the circumferential direction of the annular part 71. With respect to the number of the guide parts 72, in one embodiment, the number of the guide parts 72 is N, wherein N is 2, 3, 4, 5 or 6, and the corresponding central angle of the connection side 723 of each guide part 72 is 360°/N.

With respect to the extension trend of the guide part 72, referring to the embodiments shown in FIGS. 2a and 3b, in the expanded state, the guide part 72 uniformly tapers from the connection side 723 to the end 721. Referring to the embodiment shown in FIG. 4a, in the expanded state, the guide part 72 is first narrowed and then enlarged and finally narrowed again from the connection side 723 to the end 721. It should be noted that the extension trend is discussed referring to the shape of the projection of the guide part 72 in the radial direction of the annular part 71. The uniform tapering trend mentioned in this embodiment refers to the overall trend, while protrusion(s) or recession(s) can be locally located on the edges of the guide part 72. From other perspectives, the extension trend of the guide part 72 may be different. Referring to the embodiments shown in FIG. 2d, FIG. 3c and FIG. 4b, the guide part 72 extends from the annular part 71 in the axial direction of the annular part 71, which first expands outward in the radial direction of the annular part 71 until the maximum outer diameter 7281, and then gradually tapers.

As can be easily understood from the above, a gap is defined between the ends 721 of the guide parts 72. In one embodiment, an opening 724 is defined between two adjacent guide parts 72. In the compressed state, the two adjacent guide parts 72 contact with each other and the opening 724 is closed. During the transformation of the stent 70, the size of the opening 724 changes simultaneously.

Specifically, in one embodiment, both the guide parts 72 and the annular part 71 have hollowed cell structures. In the axial direction of the annular part 71, there are a plurality of circles of cells in the annular part 71, and the junction of the guide parts 72 and the annular part 71 is formed by the vertices of one of the circles of cells.

The cell structures of the guide part 72 and the annular part 71 can facilitate the transformation of the stent 70, and also, the mechanical properties of the stent 70 can be finely adjusted through the arrangement of the cell structures. In one embodiment, the axial length of the annular part 71 includes 1 to 2.5 cells. As shown in the figure, the axial length of the annular part 71 includes 2 cells. The specific structure of the cell may be polygonal, for example, rhombic as shown in the figure.

In one embodiment, both the guide part 72 and the annular part 71 have hollowed cell structures. The cells in the guide part 72 are divided into relative sparse areas 725 and dense areas 726. At least part of the sparse areas 725 is adjacent to the opening between two adjacent guide parts 72. Cells with different densities allow fine adjustment of the mechanical properties of different portions of the guide part 72, thereby improving the compliance of the guide part 72 and providing a smoother operating experience during the release and retrieval process.

Specifically, with reference to the embodiments shown in FIG. 2f, FIG. 3a and FIG. 4c, the first end of the annular part 71 is formed by ribs with peaks and valleys. Each cell section includes at least 3 to 7 peaks. The peaks are directly connected to the guide parts 72. In the outflow direction, the guide part 71 includes a second region and a first region.

The first region 7271 corresponds to the retrieval cell 722, which is used for the pulling wire 21 to pass through. The second region 7272 includes a second cell 7351, and the second cell 7351 is used to fix ears 7352 of two adjacent leaflets 73. The second cell 7351 has a top end and a bottom end, the top end is connected to the bottom end of the retrieval cell 722, and the bottom end is connected to the peak of the corresponding cell section, which peak is positioned centrally in the circumferential direction of the corresponding cell section.

Specifically, the guide part 72 includes four closed regions 727:
a first region 7271 located at the most proximal end of the stent for connecting with the delivering system. Here, the first region corresponds to the retrieval cell 722, including one single first cell;
a second region 7272 which corresponds to the dense area 726, in the circumferential direction of the annular part 71, the second region 7272 is aligned with the first region 7271 and serves as the central region 7275 of the guide part 72, and the second cell 7351 is the second region;
a third region 7273 which corresponds to the sparse area 725 and is located on one side of the central region 7275 in the circumferential direction of the annular part 71. In this embodiment, the third region only includes one single third cell. In other embodiments, the third region may include a plurality of cells; and
a fourth region 7274 which corresponds to the sparse area 725 and is located on the other side of the central region 7275 in the circumferential direction of the annular part 71. In this embodiment, the fourth region only includes one single fourth cell. In other embodiments, the fourth region may include a plurality of cells.

The third and fourth regions are symmetrically distributed on two sides of the second region, and have the same number and size of cells. Among the first, second, third, and fourth cells, the third and fourth cells have the same size with the largest area, the first cell has the smallest area, and the area of the second cell is between those of the first and third cells.

The dense area 726 of the second region 7272 can be implemented by increasing the number of cells within the same area, or by reducing the corresponding area with the same number of cells. In any case, the dense area 726 includes more ribs per unit area. In one embodiment, the cell area of the dense area 726 is 0.3 to 0.8 times the cell area of the sparse area 725. In a specific product, the cell area of the dense area 726 is 0.4 to 0.6 times the cell area of the sparse area 725.

Referring to the embodiment shown in FIG. 2f, the second region 7272, the third region 7273 and the fourth region 7274 each corresponds to only one cell, and one vertex of each cell is connected to the annular part 71. As shown in the figure, the cells of the third region 7273 and the fourth region 7274 have the same or similar shape, which is different from the cell shape of the second region 7272 so as to realize the different densities. In this embodiment, the cells in the third region and the fourth region have the largest areas relative to the cells in other regions of the guide part.

Referring to the embodiment shown in FIG. 2f, the guide part 72 extends axially from the annular part 71 in the axial direction of the annular part 71, which first expands outward in the radial direction of the annular part 71 until the maximum outer diameter 7281, and then gradually tapers. The maximum outer diameter 7281 is adjacent to the junction of the first region 7271 and the second region 7272. Referring to the embodiment shown in FIG. 2f, in the guide part 72, adjacent to the opening is an edge strut 7282. One end of the edge strut 7282 is connected to the retrieval cell, and the other end is connected to the annular part 71. No more than two intersection points are presented between the edge strut 7282 and the other struts of the cylindrical structure. As shown in the figure, there is only one intersection point. Referring to the embodiment shown in FIG. 2f, the edge strut 7282 is connected to a branch strut 7283 at the portion adjacent to the first end 711. The branch strut 7283 and the edge strut 7282 are respectively connected to different cell vertices at the first end 711.

In this regard, the stents 70 in FIGS. 3a to 3f and FIGS. 4a to 4e are similar and will not be repeated again.

For some stents of large sizes, for example, having a length in the range of 45 to 46 mm and a maximum outer diameter in the range of 32 to 35 mm, the guide part 71 of the stent is difficult to be compressed. Therefore, the area and location of the sparse area 725 need to be further optimized. In another embodiment as shown in FIGS. 2h to 2j, the sparse area 725 further extends to the end of the guide part 72. Specifically, referring to the embodiment shown in FIGS. 2k to 2m, the guide part 72 includes six closed regions 727.

This embodiment has a structure similar to that of the embodiment shown in FIG. 2a, including an annular part 71a and a guide part 72a. The difference is that the annular part 71a spans two cells in the axial direction, and in the second region, the inflow end node of the first cell is the same as the outflow end node of the second cell, and the leaflets are directly sutured to the second cell. In addition, the outflow end of the retrieval cell 722 is further tapered.

The embodiment shown in FIGS. 2k to 2m includes an annular part 71b and a guide part 72b.

The guide part 72b includes four regions, which are:
a first region which corresponds to the sparse area and is located at the most proximal end of the stent for connecting with the delivering system. Here, the first region corresponds to the retrieval cell, including one single first cell 81;
a second region 82 which corresponds to the dense area, in the circumferential direction of the annular part 71b, the second region is aligned with the first region and serves as the central region of the guide part 72b. The second region is used to fix ears of the valve and includes at least one second cell 82a. In this embodiment, the second region includes a plurality of cells, namely the second cell 82a and cells 82b and 82c distributed on two sides of the second cell 82a. In the outflow direction of the stent, the outflow end node of the second cell and the inflow end node of the first cell share a common end node; and
a third region 83 and a fourth region which correspond to the sparse areas and are symmetrically distributed on two sides of the second region in the circumferential direction of the annular part 71. The third region includes at least one cell. In this embodiment, the third region includes a third cell 84a, a cell 84b and a part of the cell 84c, and is adjacent to the first and second regions. The third cell 84a has the largest area among all cells of the guide part. The cell 84c spans the guide part and the annular part longitudinally.

In this embodiment, among the first, second, and third cells, the third cell has the largest area, the second cell has the smallest area, and the area of the first cell is between those of the third cell and the second cell.

The annular part includes a plurality of rows of cells, and the size of the cells decreases in the direction towards the guide part.

Only one cell is provided in each region, and one vertex of each cell in the second, third, and fourth regions is connected to the annular part 71. The cells in the sparse area 725 have approximately the same area, and the cells in the dense area 726 have approximately the same area. The cell area of the dense area 726 is 0.15 to 0.5 times, for example, 0.25 times the cell area of the sparse area 725.

At the inflow end of the stent, that is, on the inflow peaks of the inflow cell section, radiopaque element(s) is provided. For example, precious metal can be locally embedded in the inflow peaks or the inflow peaks can be consisted of precious metal. The precious metal can be distinguished from other portions by X-ray or other means of detection.

The throughout control of the stent 70 is based on the retrieval cell 722. Referring to the embodiment shown in FIG. 2g, the retrieval cell 722 has a pulling section 7221 on the side away from the annular part 71, and the pulling section 7221 is arc-shaped.

With respect to the arrangement of the pulling section 7221, in one embodiment, the pulling section 7221 and a transition section 7222 enclose one or more closed spaces. In terms of the size of the closed space, the radius of the circumscribed circle of the retrieval cell 722 is greater than or equal to twice the diameter of the strut of the retrieval cell 722. Such arrangement means that the retrieval cell 722 individually occupies a certain space, instead of an opening defined in the strut. Furthermore, both the guide part 72 and the annular part 71 have hollowed cell structures. On the circumferential surface of the stent 70, the projection area of the closed space is greater than or equal to the projection area of a single cell structure. In case of a plurality of closed spaces, the projection area of the closed space mentioned above refers to the total area of all of the closed spaces. In this embodiment, the projection area of the closed space is at least 2 square millimeters to 25 square millimeters.

In one embodiment, within the guide part 72, the edge strut 7282 is adjacent to the opening. One end of the edge strut 7282 is connected to the retrieval cell and the connection portion is adjacent to the pulling section 7221, the other end of the edge strut 7282 is connected to the annular part 71. In the figure, the retrieval cell 722 has a transition section 7222 shared with the surrounding cells on the side close to the annular part 71. The transition section 7222 is V-shaped, with its apex facing the annular part 71.

Regarding the overall shape of the stent 70, with reference to the embodiments shown in FIGS. 2a, 3a and 4a, the stent 70 generally has a cylindrical structure, and the cylindrical structure has a diameter-reduced waist 701 at the axially middle area thereof. In the figures, the interface between the guide part 72 and the annular part 71 is located at the waist 701. Further, the interface between the guide part 72 and the annular part 71 is adjacent to the minimum outer diameter of the waist 701. In the embodiments shown in the figures, the interface between the guide part 72 and the annular part 71 is located at the minimum outer diameter of the waist 701. Between two adjacent guide parts 72 is an opening 724. The opening 724 starts from the waist 701 and is further enlarged and opened away from the annular part 71. Referring to the embodiment shown in FIG. 4b and FIG. 4c, all the leaflets 73, when closed, intersect with each other on the axis of the annular part 71, and the intersection point is adjacent to the waist 701 in the axial direction of the annular part 71.

Within a circle of cells at the second end of the annular part 71, each cell is quadrangular, and the two sides near the second end are longer than the two sides near the first end, which is more convenient for radial compression of the end during retrieval or loading.

Specifically, as shown in FIGS. 4a to 4e, there is a plurality of leaflets 73 which cooperate with each other in the blood flow channel 75 to open or close the blood flow channel 75. The edge of the leaflet 73 includes a fixed edge 731 fixed to the stent 70 and a free edge 732 that cooperates with other leaflets 73 to control the blood flow channel 75. Depending on the blood flow direction controlled by the leaflets 73, the first end 711 refers to the outflow end 733 and the second end 712 refers to the inflow end 734. The two ends of the fixed edge 731 are located between the two adjacent guide parts 72, and the middle portion of the fixed edge 731 extends to the annular part 71. The two ends of the free edge 732 are respectively intersected with the stent 70, and then combined with the adjacent leaflets through the ears that further extend toward the outflow end and are further fixed to the second cells to strengthen the connection.

Referring to the figures, in the circumferential direction of the annular part 71, each guide part 72 has an axis of symmetry 7284 of its own structure, and the fixed edges 731 of two adjacent leaflets 73 meet at the axis of symmetry 7284 of the corresponding guide part 72. Viewed from another perspective, the midpoint of the fixed edge 731 is adjacent to the axial middle of the annular part 71.

Referring to the embodiment shown in FIGS. 3a to 3f, the inner side of the stent 70 is provided with an inner covering film 74. The inner covering film 74 is located on the inflow end 734 of the leaflets 73 and connected with the fixed edges 731 of the leaflets 73. In detail, the inner covering film 74 extends from the leaflets 73 to the second end 712 of the annular part 71.

The stent 70 is further provided with a perivalvular leakage preventing component 741 located on the inflow end 734 of the leaflets 73. The perivalvular leakage preventing component 741 can be exposed out of the outer circumferential surface of the stent 70. Alternatively, an outer covering film can be provided to cover the perivalvular leakage preventing component 741, that is, the inner covering film 74 and the outer covering film cover the perivalvular leakage preventing component 741 in the radial direction of the cylindrical structure.

The perivalvular leakage preventing component 741 can be in the shape of a long strip and surround the outer periphery of the stent 70. Alternatively, as shown in FIG. 3e, it can be consisted of a plurality of spaced pieces fixed to the corresponding hollowed areas of the cylindrical structure. The dotted lines in the figure are mainly used to represent the extension trend of the surface of the perivalvular leakage preventing component 741.

In another embodiment, as shown in FIG. 4f to FIG. 4h, the perivalvular leakage preventing component 741 is fixed on the outside of the inner covering film 74, and the perivalvular leakage preventing component 741 is consisted of a plurality of spaced pieces matching the corresponding cells in shape. For example, each cell corresponds to a piece of the perivalvular leakage preventing component. The perivalvular leakage preventing component 741 and the inner covering film 74 are formed in one single piece. In the expanded state, the perivalvular leakage preventing component 741 extends outward from the corresponding cell in the radial direction of the stent.

The one single piece can be formed by infiltration and fusion. The inner covering film is made of PET material, and the perivalvular leakage preventing component 741 is made of porous material, such as PU material. The holes can be the structural gaps of the inner covering film material or formed by additional processing. In the radial direction of the stent, the inner surface of the inner covering film is provided with a coating to ensure the sealing effect and close the gaps. As shown in FIG. 4f, the perivalvular leakage preventing component 741 includes a first circle of perivalvular leakage preventing component 7411, which covers the entire of each cell, a second circle of perivalvular leakage preventing component 7412 adjacent to the inflow end 734 of the first circle of perivalvular leakage preventing component 7411, and a third circle of perivalvular leakage preventing component 7413 adjacent to the outflow end 733 of the first circle of perivalvular leakage preventing component 7411. The axial length of the second circle of perivalvular leakage preventing component 7412 and the third circle of perivalvular leakage preventing component 7413 respectively corresponds to the half of each cell.

As shown in FIG. 4g, the perivalvular leakage preventing component 741 includes a first circle of perivalvular leakage preventing component 7411, and a second circle of perivalvular leakage preventing component 7412 adjacent to the inflow end 734 of the first circle of perivalvular leakage preventing component 7411.

As shown in FIG. 4h, the perivalvular leakage preventing component 741 includes a first circle of perivalvular leakage preventing component 7411, and a third circle of perivalvular leakage preventing component 7413 adjacent to the outflow end 733 of the first circle of perivalvular leakage preventing component 7411.

As for one piece of the perivalvular leakage preventing component 741, it gradually becomes thicker from the outflow end 733 toward the inflow end 734, and then gradually becomes thinner after reaching the highest protrusion (in the radial direction of the stent). The thickness variation facilitates retrieval of the highest protrusion. The highest protrusion is closer to the inflow end. It can be seen from the figure that the distance between the highest protrusion of the perivalvular leakage preventing component 741 and the inflow end of the corresponding cell is S 1. The distance between the highest protrusion of the perivalvular leakage preventing component 741 and the outflow end of the corresponding cell is S2. The ratio of S 1 : S2 ranges from 0 to 0.8, and preferably, from 0.3 to 0.8 in a specific product. The perivalvular leakage preventing component 741 can fill the space surrounded by the struts of the corresponding cell in area. The perivalvular leakage preventing component 741 is protruded to be in close contact with the side edges of the struts of the cell. That is, the lowest position of the perivalvular leakage preventing component 741 is not lower than the outer circumferential surfaces of the struts of the cell, so as to avoid gap(s) between the perivalvular leakage preventing component 741 and the side edges of the struts, which gap(s) may reduce the deformation of the perivalvular leakage preventing component 741 and thus affect the blocking effect. The side edge of the strut refers to the side of the strut facing the interior of the corresponding cell. In some cases, the perivalvular leakage preventing component can be higher than the outer circumferential surface of the stent in the expanded state.

Referring to FIGS. 5a to 5c, the catheter component includes:
an inner sheath 30 having opposing distal and proximal ends, the distal end of the inner sheath 30 is provided with a locking portion;
an outer sheath 50 slidably surrounding the outside of the inner sheath 30, the interventional instrument can be loaded and received in the outer sheath 50 in a compressed state; and
an inner core 10 slidably arranged in the inner sheath 30. One end of the inner core 10 is an extension section 11 extending out of the distal end of the inner sheath 30. The radial gap between the inner core 10 and the inner sheath 30 is a threading passage 12. The pulling wire 21 is movably arranged in the threading passage 12. A lock member 13 at the distal side of the locking portion is fixed on the extension section 11. The lock member 13 has a locked position, in which the lock member 13 is engaged with the locking portion, and an unlocked position, in which the lock member 13 is disengaged from the locking portion. In the locked state, the pulling wire 21 is wounded around the interventional instrument and then around the lock member 13 which is inserted into the locking portion to prevent the pulling wire 21 from being disengaged. The distal portion of the inner sheath 30 is fixed with a lock seat 31, and the lock seat 31 is provided with the locking portion. The locking portion is a locking hole or a locking groove.

The relative movement between the inner core 10 and the inner sheath 30 can cause the relative movement between the lock seat 31 and the lock member 13, thereby changing the constraint on the pulling wire 21. The pulling wire 21 can affect the movement of the interventional instrument 90, especially during the release process of the interventional instrument 90, so that the interventional instrument 90 can be released by stages through the pulling wire 21. Further, the sheathes that can move relative to each other provide structural basis for the throughout release and retrieval process of the interventional instrument 90, thereby providing a controllable interventional therapy, improving the therapy effect as well as the patient experience.

Here, the pulling wire 21 mainly cooperates with the retrieval cell 722 to control the interventional instrument. The structural compliance of the interventional instrument during the state switching process is improved by optimizing the structure of the guide part 72, thereby avoiding problems occurred in the throughout retrieval process of the exiting interventional instruments.

The interventional instrument 90 can be a heart valve prosthesis or a vascular stent, etc. The heart valve prosthesis can include a stent and valve leaflets connected to the stent to control blood flow. The number of leaflets is generally two or three. As required, skirt(s) can be added to the inside and/or outside of the stent. The stent itself can be formed by braiding or cutting a tube. A heart valve prosthesis can be used to replace a diseased valve in the heart, particularly the aortic valve, for example.

The lock member 13 is locked so that the pulling wire 21 is constrained. It can be understood that the pulling wire 21 will also function on the lock member 13 and thus affect the locking effect. In order to improve the mechanical properties of the lock member 13, according to an embodiment, the lock seat 31 is provided with a locking portion for engaging with the lock member 13. In the locked state, the lock member 13 is inserted into the locking portion to limit the movement of the pulling wire 21. The locking hole 311 can limit the lock member 13 on the side of the lock member 13 sway from the fixed side of the lock member 13, thereby improving the overall mechanical performance of the lock member 13. Structurally, as shown in the figures, the locking hole 311 can be a through hole or blind hole for the lock member 13 to pass through. Alternatively, the locking hole 311 can be formed as a positioning structure, for example, a positioning recess or positioning protrusion, that can improve the positioning effect of the lock member 13. Accordingly, the structure of the lock member 13 should also be adjusted.

The lock member 13 and the locking hole 311 are engaged with each other due to the relative movement between the inner sheath 30 and the inner core 10. In one embodiment, the lock member 13 moves with the inner core 10 and has the following positions:
a release position (refer to FIG. 9c), in which the lock member 13 is disengaged from the locking hole 311 to release the pulling wire 21; and
a locked position (refer to FIG. 9 b), in which the lock member 13 is inserted into the locking hole 311 to limit the pulling wire 21.

Different positions of the lock member 13 correspond to different limits on the movement of the pulling wire in the threading passage 12. In the embodiment shown in figures, the lock member 13 functions to divide a separate constraint space in the threading passage 12. When the pulling wire 21 is located at a preset position (generally through assembly), the lock member 13 constrains the pulling wire 21. In the release position, the above-mentioned constraint space is communicated with the threading passage 12, so that the pulling wire 21 can move freely to release the interventional instrument 90.

It should be noted that the pulling wire 21 is not completely immobile when being locked by the lock member 13. The pulling wire 21 can achieve the following functions based on its own material (for example, a deformable material) or size (for example, with a long extension length): when the pulling wire 21 is constrained by the lock member 13, it can release a preset movement of interventional instrument 90 through its own deformation. With reference to the embodiment shown in FIG. 9b, through the self-deformation of the pulling wire 21, the interventional instrument 90 is semi-released even though the lock member 13 is in the locked state, where the connecting ears 92 of the interventional instrument 90 are released from the corresponding structure, but the overall profile of the interventional instrument 90 is still controlled by the pulling wire 21. The above arrangement provides a structural basis for controlling the release of the interventional instrument 90 by controlling the pulling wire 21.

It should be noted that the deformation of the pulling wire 21 mentioned above refers to the deformation in the extension path of the pulling wire 21 rather than the length change caused by material stretching. In this field, in order to control the release of the valve by the pulling wire, the stretching rate of the pulling wire should be as small as possible. Other description about the deformation of the pulling wire 21 in this disclosure should also meet this requirement.

As mentioned above, one end of the lock member 13 is limited by the locking hole 311, and the other end also requires a corresponding structure for a stable connection. In one embodiment, the extension section 11 of the inner core 10 is provided with a mounting seat 131, and there are a plurality of lock members 13 that are rod-shaped and are all fixed to the mounting seat 131 and extend proximally from the mounting seat 131. The mounting seat 131 is provided with mounting holes 132 for mounting the lock members 13. The distal end of the lock member 13 is inserted and fixed in the mounting hole 132, and the proximal end of the lock member 13 extends out of the mounting hole 132. In order to facilitate limiting the threading of the pulling wire 21, guide holes 312 can be provided in the lock seat 31 or the mounting seat 131, and the pulling wire 21 extends outward from the corresponding guide hole 312.

The engagement direction of the lock member 13 with the locking hole 311 in this embodiment is also important. It can be understood that the distal end of the lock member 13 is connected to the inner core 10, and the proximal end of the lock member 13 extends toward the lock seat 31 and engages with the locking hole 311 as it moves. The lock member 13 moves proximally (from the distal end to the proximal end) to engage with the locking hole 311, which can improve the compactness of the structure. In the embodiment shown in the figures, the lock member 13 and the mounting seat 131 in the lock seat 31 can be arranged inside the interventional instrument 90 in the loaded state, which are located in the bare stent section of the valve stent and can fill the large gap between the valve stent and the inner core in the loaded state, preventing the valve stent from collapsing or bending due to lack of support during the crimping process. Further, providing the lock member 13 at the front end can effectively reduce the interference with the pulling wire tube and the pulling wire, facilitating the control of the overall volume of the delivering system and making a series of operations convenient during the interventional therapy.

More importantly, the lock member 13 arranged in such direction makes the process of assembling the interventional instrument 90 to the delivering system more convenient. On the contrary, if a locking structure moving distally (from the proximal end to the distal end) for engagement is used, the process of assembling the interventional instrument will be more cumbersome and the interventional instrument is very easy to fall off, resulting in rework and affecting production efficiency.

The mounting hole 132 allows a stable engagement between the lock member 13 and the mounting seat 131. In a specific product, the lock member 13 can be fixedly connected with the mounting hole 132. Considering the production difficulty, bonding or welding is preferred. During the assembly, the structure of the mounting hole 132 can facilitate the assembly process and improve production efficiency. On this basis, in one embodiment, the mounting hole 132 is opened on the circumferential surface of the mounting seat 131, and the opened portion can at least accommodate the lock member 13.

The opened mounting hole 132 can facilitate operation and inspection, ensure the stability of the connection, and improve the overall stability of the device. More importantly, the opened mounting hole 132 can structurally ensure that the lock member 13 is mounted to the bottom of the mounting hole 132, avoiding the uncertainty caused by a blind hole, thereby ensuring the locking spacing between the end surface of the lock member 13 and the locking hole 311.

In terms of quantity, the lock members 13 and the locking holes 311 can be adjusted as required. In one embodiment, a plurality of lock members 13 are provided at intervals in the circumferential direction of the inner core 10, and the locking holes 311 are arranged corresponding to the lock members 13. The number of lock members 13 and locking holes 311 can be adjusted to improve the constraint on the pulling wire 21, thereby achieving multi-dimensional control of the interventional instrument 90, which is particularly significant in improving control accuracy. However, the increase in quantity will increase the complexity of the structure, which will have a certain impact on assembly and stability. Therefore, in a specific product, at least three lock members 13 are evenly arranged in the circumferential direction of the inner core 10, and the locking holes 311 are arranged corresponding to the lock members 13.

Referring to FIGS. 6a to 6c, in another embodiment, the lock members 13 are provided with a slidable push block 134, and a compression spring 135 is provided between the push block 134 and the mounting seat 131. The engagement portion between the lock member 13 and the pulling wire 21 is located at the proximal side of the push block 134.

The push block 134 is in a sheet shape and is provided with through holes for the lock members 13 to pass through. The distance between the push block 134 and the mounting seat 131 is greater than or equal to the length of the compression spring 135 in its natural state, thereby preventing the push block 134 from being separated from the lock members 13 under the action of the compression spring 135.

When operating the inner core 10 to drive the lock member 13 to engage with the guide hole 312 in the lock seat 31, after the push block 134 abuts the lock seat 31, the compression spring 135 is compressed. When it is desired to release the engagement between the pulling wire 21 and the lock member 13, the compression spring 135 returns to drive the push block 134 to push the pulling wire 21 to move proximally so that the proximal section of the lock member 13 exposed out of the push block 134 is reduced, which facilitates the disengagement between the engagement loop 22 of the pulling wire 21 and the lock member 13.

A support sleeve 317 is movably installed on the inner core 10. When the lock member 13 is in the locked position, the support sleeve 317 is in contact between the push block 134 and the lock seat 13, the compression spring deforms, and the lock member 13 extends out of the push block 134 to engage with the locking portion.

It is the pulling wire 21 that actually interacts with the interventional instrument, so the arrangement details of the pulling wire 21 also have a synergistic effect. In one embodiment, the delivering system for the interventional instrument 90 further includes a wire control tube 20. The wire control tubes 20 are arranged movably around the inner core 10. One end of the pulling wire 21 is a driving end 211 that is connected to the wire control tube 20, the other end of the pulling wire is the working end 212 (i.e., the engagement loop 22 below). When the interventional instrument is in the loaded state, the working end 212 is wound around the interventional instrument 90 and then engages with the lock member 13. There are multiple groups of pulling wires 21, and each group corresponds to one lock member 13 and consists of a single pulling wire 21 that extends in one direction and has a wire loop (i.e., the engagement loop 22) at the end, and is movably connected to the lock member 13 through the wire loop.

Alternatively, each group of pulling wire 21 consists of a single pulling wire that extends back and forth, with a wire loop formed at the turning point, and is movably connected to the lock member through the wire loop.

The wire control tube 20 functions to control the working state of the pulling wire 21, and in particular, to control the interventional instrument 90 by controlling the pulling wire 21. In this embodiment, the wire control tube 20 is movably provided to realize its own driving function. Specifically, the proximal end of the wire control tube 21 is movable relative to the control handle. In this embodiment, the proximal end of the wire control tube 21 is movable relative to the inner sheath. Regarding the specific structure of the wire control tube 20, refer to one embodiment in which the wire control tube 20 is movably disposed between the inner core 10 and the inner sheath 30. The wire control tube 20 is disposed between the inner core 10 and the inner sheath 30 to avoid interference between the wire control tube 20 and the lock seat 31, thereby providing more space for the assembly of the interventional instrument 90.

The wire control tube 20 is a tube in the embodiment shown in the figures. However, it is also possible by extending the proximal end of the pulling wire 21, that is, it is possible to achieve the above function by directly operating the proximal end of the pulling wire 21 through the control handle 60. Therefore, in a specific product, the specific form of the wire control tube 20 may be varied. The description below is mainly based on the form of the wire control tube 20 as shown in the figure. The same applies to other forms and will not be repeated again.

Referring to the above description and in combination with the embodiment of FIG. 9b, through the self-deformation of the pulling wire 21, the interventional instrument 90 is semi-released even though the lock member 13 is in the locked state, where the connecting ears 92 of the interventional instrument 90 are released from the corresponding structure, but the overall profile of the interventional instrument 90 is still controlled by the pulling wire 21. The above arrangement provides a structural basis for controlling the release of the interventional instrument 90 by controlling the pulling wire 21. In one embodiment, the section of the pulling wire 21 extending to the distal side of the fixed plate of the lock seat 31 is a controllable section that controls the release degree of the interventional instrument. When the working end 212 of the pulling wire 21 is constrained by the lock member, the length of the controllable section is adjusted through the position of the wire control tube 20.

The working state of the lock member 13 and the working state of the wire control tube 20 can be linked or independent of each other. In case where the two are independent of each other, when the lock member 13 is not unlocked, the wire control tube 20 can control the constrained pulling wire 21 to adjust its state through its own movement.

Regarding the specific structure of the lock seat, refer to one embodiment in which the lock seat 31 includes a guide plate 313, a connecting sleeve 314 and a fixed plate 315 connected in sequence from the distal end to the proximal end. The locking hole 311 is opened in the fixed plate 315, and the guide hole 312 is opened in the guide plate 313 and corresponds to the locking hole 311 in position.

The lock member 13 passes through the guide hole 316 in the locked position, and is inserted into the corresponding locking hole 311 after passing over/across the periphery of the connecting sleeve 314. The section of the lock member 13 located over/across the periphery of the connecting sleeve 314 serves as the working section, and the working end 212 of the pulling wire 21 is constrained on this working section.

The guide plate 313 and the fixed plate 315 define a relatively small and closed space in the threading passage 12, which can effectively improve the stability of the lock member 13 with respect to the pulling wire 21. Also, under the action of the guide hole 316, the mechanical properties of the lock member 13 can be effectively improved, ensuring that the interventional instrument 90 with high elasticity can be controlled.

In addition to providing guidance for the lock member 13, the lock seat 31 can also provide guidance for the pulling wire 21. In the embodiment as shown in FIG. 5c, the fixed plate 315 is provided with guide holes 312 for the pulling wires 21 to pass through. The pulling wire 21 extends from the wire control tube 20 through the guide hole 312 to the distal side of the fixed plate 315, and engages with the lock member 13.

For connection with the wire control tube 20, the pulling wire 21 needs to pass through the fixed plate 315 of the lock seat 31. That is to say, the pulling wire 21 engages with the lock member 13 through the guide hole 312. The guide hole 312 can guide the pulling wire 21, thereby achieving stable driving of the pulling wire 21 by the wire control tube 20. Furthermore, the guide hole 312, depending on its own position, can adjust the force application point of the pulling wire 21 on the interventional instrument 90, thereby better controlling the interventional instrument 90. In one embodiment, the lock seat 31 is provided with locking holes 311 that engage with the lock members 13. There are a plurality of guide holes 312 and locking holes 311, which are alternately arranged in the circumferential direction of the fixed plate 315. The adjacent guide hole 312 and locking hole 311 can not only adjust the force application point of the pulling wire 21 on the interventional instrument 90 as mentioned above, but also facilitate a compact arrangement of the various structures of the lock seat 31 while avoiding undesired interference between adjacent pulling wires 21 (if there are a plurality of pulling wires 21).

The constraint on the pulling wire 21 is actually determined by three factors, namely how the proximal end of the pulling wire 21 is related to the control handle (in this embodiment, the proximal end of the pulling wire 21 is connected to the wire control tube), how the working end 212 of the pulling wire 21 interacts with the interventional instrument 90, and how the working end 212 of the pulling wire 21 interacts with the lock member 13, which will be exemplarily explained below.

Regarding how the working end 212 of the pulling wire 21 is related to the interventional instrument 90, with reference to an embodiment, the pulling wire 21 and the interventional instrument 90 are configured as follows:
the working end 212 of the pulling wire 21 engages with the lock member 13 after passing through the structure of the interventional instrument 90; or
the interventional instrument 90 is provided with a connector 91, and the working end 212 of the pulling wire 21 passes through the connector 91 and then engages with the lock member 13.

This embodiment includes the two implementation methods.

The working end 212 of the pulling wire 21 engages with the lock member 13 after passing through the structure of the interventional instrument 90 (refer to FIG. 5f). The structure of the interventional instrument 90 can be a hollow portion of the stent of the interventional instrument 90, or a hole opened in the stent of the interventional instrument 90, or a portion formed by extending the material of the interventional instrument 90 itself. In one embodiment, the interventional instrument is provided with a space (such as the hollow portion mentioned above) or an additional threading hole (such as the retrieval cell mentioned above).

The working end 212 of the pulling wire 21 extends in the direction of the interventional instrument 90, passes through the hole at the end of the interventional instrument 90, extends in the direction of the lock seat 31, and is finally wounded around the locking rod between the guide plate 313 and the fixed plate 315. The advantage of this embodiment is that it can realize direct control of the interventional instrument 90 by the pulling wire 21 and has a small number of components, which facilitates production and assembly while reducing the possibility of component failure.

Regarding the specific engagement between the pulling wire 21 and the lock member 13, refer to one embodiment in which one end of the pulling wire 21 that engages with the lock member 13 (i.e., the working end 212) is provided with an engagement loop 22, and the lock member 13 passes through the engagement loop 22 to constrain the pulling wire 21.

The lock member 13 in the locked position passes through the closed space enclosed by the pulling wire 21 to constrain the pulling wire 21. The closed space can be formed by the threading of the pulling wire 21 or provided by the engagement loop 22.

Regarding the formation of the engagement loop 22, according to an embodiment, the engagement loop 22 can be a separate component or is formed by winding the pulling wire 21. The separate component allows the use of different materials. For example, in some embodiments, the engagement loop 22 is made of a radiopaque material to facilitate real-time control of the intervention process. The method by winding the pulling wire 21 involves a simple and stable structure, which facilitates production and assembly.

Regarding the specific form of winding, the pulling wire 21 can be a single wire extending from the driving end 211 to the working end 212, and the single wire is wound at the working end 212 to form the engagement loop. Alternatively, the pulling wire 21 has a double-wire structure, which is folded back at the working end 212, and the folded portion forms the engagement loop.

In order to drive the pulling wire 21, the catheter component further includes a wire control tube 20. The wire control tube 20 is arranged around the outside of the inner core 10. One end of the pulling wire 21 is the driving end 211 which extends proximally in the wire control tube 20 and is controlled by the control handle, and the other end of the pulling wire 21 is the working end 212 (i.e., the engagement loop 22 below). When the interventional instrument is in the loaded state, the working end 212 engages with the lock member 13 after winding around the interventional instrument 90.

Compared with the above embodiments, the wire control tube 20 in this embodiment provides an independent movement space for the pulling wire 21, to prevent the pulling wires 21 from interfering with each other during the movement. The proximal end of the pulling wire 21 can be directly controlled by the control handle, or can be connected to the control handle through an intermediate component.

The outer sheath 50 can cover or release the interventional instrument 90 through its movement relative to the inner core 10. With the cooperation of the pulling wire 21 mentioned above, the outer sheath 50 can retrieve the interventional instrument 90, thereby realizing a controllable release process of the interventional instrument 90.

As shown in FIGS. 7a to 7h, the control handle has opposing distal and proximal ends for driving a plurality of controlled components to move relative to each other. The handle includes a support body 61 and a plurality of connection components installed on the support body 61. Relative to the support body 61, the connection components include fixed and movable connection components. The movable connection components, depending on the transmission mode, involve thread transmission, rack-and-pinion transmission, and direct transmission, and the connection components in each transmission mode are arranged in sequence from the distal end to the proximal end.

The controlled components can be all configured as sheathes which are in insertion fit, or not sheathes, but only extend proximally to the control handle. The controlled components can move relative to the control handle, or one or more of them can be fixedly positioned relative to the control handle.

Thread transmission, rack-and-pinion transmission, and direct transmission each have their own advantages. The arrangement in sequence from the distal end to the proximal end of the control handle 60 can meet the different movement requirements of the sheathes and utilize the spatial structure of the control handle 60 for structural optimization, thereby improving the driven effect of the controlled components within a certain volume of the control handle 60, which will be explained below in conjunction with the specific structure.

The control handle 60 includes a thread fitting area 62 located on the distal side and a rack-and-pinion fitting area 63 located on the proximal side in the axial direction. The connection components by thread transmission and rack-and-pinion transmission are respectively arranged in the corresponding fitting areas.

Thread fit and rack-and-pinion fit are characterized by their ability to provide stable transmission ratios, especially when the controlled components require precise control. The advantage of the thread fit is that rotation in the circumferential direction of the control handle 60 can be achieved, which facilitates to save the circumferential space of the handle, but still requires a certain axial space. On the other hand, the advantage of the rack-and-pinion fit is that it can save the axial space of the handle, but still requires a certain circumferential space. In this embodiment, the arrangement of the control handle 60 is optimized by complementing the advantages and disadvantages of the two fit forms, while ensuring the driving effect. In a specific product, the ratio of the axial length of the thread fitting area 62 to the axial length of the control handle 60 is greater than or equal to 0.4. It is further preferred that the ratio of the axial length of the thread fitting area 62 to the axial length of the control handle 60 is greater than or equal to 0.55.

In this embodiment, the thread fitting area 62 is provided with a thread connection component 621, which includes:
a driving ring 622 rotatably surrounding the outer circumference of the support body 61; and
a clamping member 625 slidably installed on the support body 61 for connecting with the controlled component (specifically, the outer sheath 50). The outer circumferential surface of the clamping member 625 and the inner circumferential surface of the driving ring 622 are in a thread fit.

The clamping member 625 has a mounting hole, and the proximal end of the outer sheath 50 is inserted into the mounting hole and is clamped and fixed by the wall of the mounting hole. Additionally, glue or fastener can be used to prevent loosening.

The outer surface of the driving ring 622 is provided with a friction surface for the operator to hold. As an active component, the driving ring 622 is rotatably installed on the support body 61 to drive the clamping member 625. The support body 61 can be provided with an axial groove and the clamping member 625 is slidably disposed in the axial groove. The clamping member 625 is at least partially provided with a male thread structure for engaging with the driving ring 622 in a transmission manner. The clamping member 625 is mainly used to fix the proximal end of the controlled component. When the controlled component is a sheath, it is preferred that the clamping member 625 is connected with the sheath in a sealing manner.

The controlled component needs to be positioned after moving to an appropriate position to avoid being affected by other operations. In one embodiment, the support body 61 is surrounded by a slidable positioning ring 623 on the outer periphery thereof, and the driving ring 622 has a positioned state in which the driving ring 622 engages with the positioning ring 623 and a free state in which the driving ring 622 is separated from the positioning ring 623. In the positioned state, the positioning ring 623 limits the rotation of the driving ring 622.

The positioning ring 623 is a structure that is slidably installed on the support body 61 and can position or release the driving ring 622 by changing its position.

It should be noted that the positioning ring 623 should prevent itself from rotating relative to the support body 61 while sliding on the support body 61. Therefore, a guide groove/slide rail can be provided on the support body 61. The positioning ring 623 and the guide groove/slide rail can be engaged with each other in an interference fit to ensure friction between the two and avoid the failure of the restriction of the positioning ring 623 on the driving ring 622 or an accidental locking affecting the operator's therapy.

Referring to FIG. 7b, positioning teeth 624 are provided on the side of the driving ring 622 facing the positioning ring 623. The driving ring 622 meshes with the positioning teeth 624 in the positioned state. The advantage of this arrangement is that it can realize the function of the positioning ring 623 while reducing the impact on the movement of the driving ring 622, as well as optimizing the arrangement of the components of the control handle 60.

Referring to FIGS. 7b to 8, the connection component in the rack-and-pinion fitting area 63 is a pinion connection component 631 which is disposed close to the proximal end of the support body 61. The pinion connection component 631 can improve the utilization of the axial space of the handle. Considering the operator's ergonomics, arranging the pinion connection component 631 on the proximal side of the handle can provide a better operating experience. Further, the structure of the pinion connection component 631 in the circumferential space can improve the drive on the above thread connection component 621 by the operator, and the two cooperate with each other to further improve the operating experience.

Regarding the specific structure of the pinion connection component 631, according to an embodiment, a group of pinion connection component 631 is provided that is disposed close to the proximal end of the support body 61 and includes:
a rack 634 movable in the axial direction of the support body 61 and provided with a clamping seat 633 for sealing connection with the corresponding controlled component (specifically, the wire control tube 20); and
a pinion 635 that is rotatably mounted on the support body 61 and meshes with the rack 634.

The clamping seat 633 has a mounting hole. The proximal end of the wire control tube 20 is inserted into the mounting hole and is clamped and fixed by the wall of the mounting hole. Additionally, glue or fastener can be used to prevent loosening.

In this embodiment, the pinion 635, as an active component, can drive the rack 634 to move to drive the controlled component. At least a part of the pinion 635 extends to the outside of the support body 61 or is provided with a corresponding structure extending to the outside of the support body 61, in order to facilitate operation and assembly and utilize the peripheral space of the support body to improve space utilization.

In a specific product, the rack 634 can be provided with a corresponding structure to improve cooperation with the controlled component. In one embodiment, the control handle 60 includes a support cylinder 638 that surrounds the proximal end of the support body. The controlled component and the pinion is connected based on the following arrangement provided in the support body 61:
a base 632 slidably installed inside the support cylinder 638, on which the rack 634 is fixedly mounted; and
a clamping seat 633 installed on the base 632 and used to connect the proximal end of the wire control tube 20.

The base 632 functions to provide stable motion constraint, the clamping seat 633 can realize the positioning of the wire control tube 20 and the application of driving force, and the rack 634 is used to bear the driving force from the pinion 635.

The clamping seat 633 can be fixedly installed on the base 632. Alternatively, the clamping seat 633 can have a slight axial movement margin relative to the base 632. For example, a guide structure (a slide rail 6321 shown in FIG. 7f) can be provided between the clamping seat 633 and the base 632 for guiding the axial movement of the clamping seat 633 and can prevent the clamping seat 633 from radially disengaging from the base 632. The base 632 is provided with a first limiting protrusion 6341 and a second limiting protrusion located on two sides of the clamping seat 633. The distance between the first limiting protrusion 6323 and the second limiting protrusion 6324 is slightly larger than the clamping seat 633, so that the clamping seat 633 can slide slightly. The second limiting protrusion 6324 is configured as a resilient snap 6322 to facilitate the installation of the clamping seat 633.

Similar to the thread connection component 621 above, the controlled components need to be positioned after moving to an appropriate position to avoid being affected by other operations. In one embodiment, the pinion 635 is linked with a driving portion 636 that extends radially to the outside of the support cylinder. The driving portion 636 is movably installed on the support cylinder 638. Specifically, the driving portion 636 can rotate relative to the support cylinder 638 to drive the pinion 635 to rotate, and the driving portion 636 can slide relative to the support cylinder 638. Further, a connection locking mechanism 637 is provided between the two to limit the rotation of the driving portion 636.

Referring to FIGS. 7d to 8, the support cylinder 638 is partially radially extended to form an outer protrusion 6382, and the driving portion 636 is disposed outside the outer protrusion 6382 and is linked with the pinion 635. The driving portion 636 is generally a hollow knob and covers the outer protrusion 6382. The connection locking mechanism 637 includes:
first locking teeth 6371 provided on the driving portion 636;
second locking teeth 6372 engaged with the first locking teeth 6371 and arranged on the outer protrusion 6382; and
a maintaining component 6373 for maintaining the first locking teeth 6371 and the second locking teeth 6372 in an engaged state to limit the rotation of the driving portion 636.

The pinion 635 is fixedly connected to the driving portion 636 and can slide relative to the support cylinder 638 in its axial direction so as to achieve meshing or separation of the first locking teeth 6371 and the second locking teeth 6372. It should be noted that the rotation axis of the driving portion 636 is collinear with the rotation axis of the pinion 635 and extends substantially in the radial direction of the control handle. The sliding direction of the driving portion 636, when switching positions, is consistent with the direction of the rotation axis of the driving portion 636.

The pinion 635 is always meshed with the rack 634 during the sliding process. That is, the pinion 635 is always meshed with the rack 634 during its own sliding stroke, which can be achieved, for example, by providing the rack 634 with a meshing width larger than that of the pinion 635.

According to an embodiment, the maintaining component 6373 functions to maintain the relative position of the driving portion 636 and the outer protrusion 6382, thereby maintaining the locking effect. Specifically, the maintaining component 6373 includes:
two snapping posts 6374 that are slidably installed on the driving portion 636 and can move close to or far away from each other in the radial direction of the pinion 635;
a snapping block 6381 provided on the outer protrusion 6382; and
a reset member 6376 disposed in the driving portion 636 and acting on the two snapping posts 6374 to keep them away from each other.

When the two snapping posts 6374 are far away from each other, the end surface of the snapping post 6374 abuts one of the end surfaces of the snapping block 6381 to keep the connection locking mechanism 637 in a locked or unlocked state. When the two snapping posts 6374 are close to each other, the snapping post 6374 can overcome the constraint of the snapping block 6381 to realize the state switching of the connection locking mechanism 637.

The maintaining component 6373 actually functions as a secondary lock member to ensure the stability of the connection locking mechanism 637.

According to an embodiment, the pinion 635 has a rotation shaft 6351 connected to the driving portion 636. The support cylinder 638 is fixedly provided with a limiting seat 6383 that surrounds the outside of the rotation shaft 6351. The limiting seat 6383 can abut the end surface of the pinion 635 to prevent the driving portion 636 and the pinion 635 from being separated from the support cylinder 638.

According to an embodiment, the support body 61 is fixedly installed with a first fixed seat 611 fixedly connected to the inner sheath 30. The first fixed seat 611 is located between the clamping member 625 and the clamping seat 633 in the axial direction of the support body. The proximal end of the support body 61 is slidably mounted with a sliding seat 612 fixedly connected to the inner core 10.

The control handle 60 is provided with an exhaust component 64 as a fixed connection component, and the corresponding controlled component is proximally communicated with and fixed to the exhaust component. The proximal end of the protection sheath 51 is fixedly connected to the exhaust component 64.

The air in the radial gap between the protection sheath 51 and the outer sheath 50 can be evacuated to the distal end by injecting liquid (for example, physiological saline) through the liquid injection hole 641 in the exhaust component 64.

It is possible to realize the emptying of the sheathes by the single exhaust component by opening holes in the walls of the sheathes in combination with relative movement. The sheathes sharing a single exhaust component 64 can effectively avoid repeat of exhaust components 64 and improve the utilization of the component in the control handle 60.

Referring to FIGS. 9 to 14, the crimping device 240 has a cylindrical structure with an axis direction. In the axis direction, the cylindrical structure includes a connection section 241, a transition section 242 and an extension section 243 from the proximal end to the distal end.

A radially deformable locking mechanism is disposed at one end of the connection section 241, which has a locked state in which the crimping device 240 and the outer sheath 50 are fixed relative to each other, and an unlocked state in which the crimping device 240 and the outer sheath 50 can be separated from each other. During the locking process, the outer sheath 50 deforms at the corresponding position (the position where the locking mechanism locks).

In this embodiment, the length of the connection section 241 is 30 to 80 mm (larger than the length of a common valve). For example, after the valve is retracted into the outer sheath 50, the length between the proximal end of the valve and the distal end of the outer sheath 50 is L3. The distance between the force application portion of the locking mechanism and the distal end of the outer sheath 50 is L4. L4 is greater than L3. The deformation (radial shrinkage) of the outer sheath 50, especially the valve-accommodation area, can be avoided and the resistance of loading the valve can be reduced.

The transition section 242 is enlarged relative to the connection section 241 and has an inner conical surface 2421 that guides the radial deformation of the valve. The transition section 242 is fixed to an end of the connection section 241 away from the locking mechanism. The valve in the expanded state can be compressed under the guidance of the inner conical surface 2421, and then loaded into the outer sheath 50 through the connection section 241. Alternatively, the stent can be partially deformed. That is, in the pre-loaded state, a part of the stent can be radially compressed by the inner conical surface 2421, or has been in the compressed state.

One end of the extension section 243 and the transition section 242 form a chamber 2431 for receiving the valve in the expanded state. The distal end of the extension section 243 has an opening 2432 for pre-loading the valve and fluid immersion among others. The crimping device 240 is made of transparent material, which facilitates observation of the valve status and the position relationship relative to the outer sheath.

The chamber 2431 has sufficient axial length, which is generally larger than the valve length, so that the valve can be fully received in the chamber even in the expanded state, avoiding long-time crimping of the valve material of the valve, which cooperates with the wire control method, so that the valve can still assume the expanded state through the release of the pulling wire even after the valve is connected with the pulling wire.

The specific arrangement of the locking mechanism can be varied. Two options are provided below. Refer to FIGS. 9 to 17. The locking mechanism includes a plurality of elastic claws 2411 and a sliding sleeve 244. The elastic claws 2411 are located at the end of the connection section 241 and spaced from each other in the circumferential direction of the connection section 241. The sliding sleeve 244 surrounds the outer periphery of the plurality of elastic claws 2411 and is slidably fitted with the connection section 241. The sliding sleeve 244 has relative fastening and release positions on its sliding path.

The sliding sleeve 244 in the fastening position gathers and exerts force on the plurality of elastic claws 2411 inward, causing the elastic claws 2411 to deform radially inward (which can be understood as the radial deformation of the locking mechanism) to press the outer sheath 50;

The distal end of the outer sheath 50 has a diameter-enlarged section for accommodating the interventional instrument, and the locking mechanism acts on the diameter-enlarged section.

The sliding sleeve 244 in the release position releases the force exerted on the plurality of elastic claws 2411. The sliding sleeve 244 is more convenient to operate.

Referring to FIGS. 15 to 17, there are protruding ribs 2412 on the outside of the elastic claws 2411. The ribs 2412 on all the elastic claws 2411 are distributed in a ring around the circumference of the connection section 241 to form a protruding ring 2413. The sliding sleeve 244 in the fastening position exerts force on the ribs 2412 through its inner wall. There are multiple protruding rings 2413. The protruding heights of the protruding rings 2413 relative to the corresponding elastic claw 2411 vary sequentially, and the farther away from the transition section 242, the higher the protruding height of the protruding ring 2413 is. Correspondingly, the inner wall of the sliding sleeve has multiple regions with gradually increasing thickness from the distal end to the proximal end, and the multiple regions are adapted to the corresponding protruding rings.

In the figure, the elastic claws 2411 correspond to a first protruding ring 2413a and a second protruding ring 2413b in the axial direction, and the first protruding ring 2413a is higher than the second protruding ring 2413b. The sliding sleeve 244 has a cylindrical structure with a constant outer diameter and a first region 2442 and a second region 2443 adapted to the first protruding ring 2413a and the second protruding ring 2413b in the axial direction. The inner diameter of the first region 2442 is greater than the second region 2443.

When the sliding sleeve 244 switches from the release position to the fastening position, the second region 2443 abuts the first protruding ring 2413a and is restricted from further sliding. A groove 2414 is formed between the two protruding rings, and a protrusion 2441 matching the groove 2414 is provided between the two regions. In the fastening position, the protrusion 2441 is received in the groove 2414 to prevent the sliding sleeve 244 from sliding proximally to switch to the release position, which avoids accidental unlocking of the crimping device during loading, and also providing locking and limiting effect.

The inner wall of the sliding sleeve 244 is provided with a plurality of friction-reducing ribs 2444, the friction-reducing ribs 2444 extend in the axial direction, and the friction-reducing ribs 2444 are distributed in the circumferential direction of the connection section 241. The friction-reducing ribs 2444 constitute the second region 2443, and the protrusion 2441 is provided at the proximal end of the friction-reducing ribs 2444.

To facilitate operation, the outer wall of the sliding sleeve 244 can be provided with an anti-slip structure. For example, there are multiple anti-slip ribs 2445 extending in the circumferential direction of the connection section 241 and spaced apart in the axial direction.

As shown in FIGS. 18 to 22, another locking mechanism includes an elastic ring 245 and a lock cap 246. The elastic ring 245 is located inside the connection section 241. The lock cap 246 is threaded with the connection section 241, and at least a part of the lock cap 246 extends into the interior of the connection section 241 and abuts the elastic ring 245. The elastic ring 245 surrounds the outer periphery of the outer sheath 50, and its end is pressed by the lock cap 246 and limited by the inner wall of the connection section 241, causing the inner ring of the elastic ring 245 to compress (which can be understood as the radial deformation of the locking mechanism) to press the outer sheath 50 tightly. The elastic ring 245 provides a gradual deformation, and the adjustment is more precise.

The end of the connection section 241 away from the transition section 242 has an installation chamber 2415 with an increased inner diameter. The elastic ring 245 is located in the installation chamber 2415, and the lock cap 246 is threaded with the inner wall of the installation chamber 2415. The inner wall of the installation chamber 2415 has a step structure 2416. In the axial direction, the elastic ring 245 is clamped between the step structure 2416 and the lock cap 246, and the rest of the inner wall of the installation chamber limits the radial outward deformation of the elastic ring 245.

In order to prevent the valve from being exposed outside the crimping device, the length of the chamber 2431 is greater than or equal to the length of the valve in the expanded state. Further, in order to facilitate pre-loading, the transition section 242 and the extension section 243 are separate structures, and the junction of the two is located at the position of the chamber 2431 adjacent to the transition section 242.

An assembly indicator 2417 (such as a score or a scribe) is provided on the outer periphery of the connection section 241 at the distal side. The assembly indicator 2417 needs to be substantially aligned with the distal end surface of the outer sheath 50.

In the axis direction, the length of the chamber 2431 is L1, the length of the transition section 242 is L2, and L2 : L1 = 2.0 to 3.0.

The maximum inner diameter of the chamber 2431 is D1, and the inner diameter of the junction of the two is D2, and D2 : D1 = 1.0 to 1.5. The extension section 243 extends with a constant diameter. In order to facilitate connection, there is an extension area with a constant diameter between the transition section 242 and the extension section 243, where D2: D1=1. Alternatively, the extension section 243 can extend from the proximal end to the distal end with an enlarged diameter, but the overall diameter enlarging trend is slower than that of the transition section 242.

An embodiment of the present disclosure also provides an interventional instrument preloading and delivering system, including:
a catheter component 260 having opposing distal and proximal ends;
a crimping device 240 having a cylindrical structure with an axis direction, in the axis direction, one end of the crimping device 240 surrounds the distal end of the catheter component 260, and the other end defines a chamber 2431 having an inner conical surface 2421;
an interventional instrument 90 including a stent with a cylindrical structure and having relative compressed and expanded states. The interventional instrument 90 is located in the chamber 2431 and is in the expanded state. The stent includes: an annular part 71, which has a radially deformable structure, and the two ends thereof in its own axial direction are a first end and a second end respectively. The edge of the first end includes a plurality of cell sections arranged in sequence in the circumferential direction of the annular part; a plurality of guide parts 72, which are arranged at intervals in the circumferential direction of the annular part 71. One side of each guide part 71 is connected to a corresponding cell section, and the other side gradually tapers to the end, where a retrieval cell 722 is provided;
a control handle 60 connected to the proximal end of the catheter component 260; and
a pulling wire 21, one end of which is controlled by the control handle 60 and the other end extends distally in the catheter component 260 and engages with the retrieval cell 722 of the interventional instrument. Relative to the interventional instrument 90, the pulling wire 21 has a locked state, in which the pulling wire 21 can pull the interventional instrument 90 to transform from the expanded state to the compressed state, and an unlocked state, in which the pulling wire 21 is disengaged from and releases the interventional instrument 90.

In this embodiment, the interventional instrument is located in the chamber and is in the expanded state, which is particularly suitable for a heart valve prosthesis device that is preserved with the valve dried, avoiding the disadvantages caused by long-time crimping after preloading in the prior art. The details of the other components can refer to the previous embodiments.

An embodiment of the present disclosure further provides an interventional instrument preloading and delivering assembly, in which a packaging component is provided in addition to the interventional instrument preloading and delivering system of the previous embodiments. The packaging component at least includes a carrying tray, and the carrying tray includes a tray body, the tray body has opposing top and bottom sides, the top side of the tray body has a recessed portion that forms an accommodation area, and the interventional instrument preloading and delivering system is located in the accommodation area. Referring to FIGS. 28 to 31, the packaging component includes a tray 1. The tray 1 includes a tray body 100, and the tray body 100 has a top side 101 and an opposing bottom side 102. The top side 101 of the tray body 100 has a recessed portion that forms an accommodation area 110 for accommodating the delivering system 2. The depth and shape of the accommodation area 110 are adapted to different parts of the delivering system 2 or meet corresponding functions. The material of the tray body 100 is PET (PETG, PETE) or PP. The recessed portion on the top side 101 of the tray body can also strengthen the structure of the tray body 100. Referring to FIG. 32, the portion of the accommodation area 110 corresponding to the interventional instrument 90 is a liquid tank 111, and the periphery of the interventional instrument 90 is spaced apart from the wall of the liquid tank. When loading the interventional instrument into the catheter component 260, the operator can pour a certain volume of ice water 1114 into the liquid tank 111 to provide a suitable temperature environment. Alternatively, other acceptable fluids can be used. The ice water can immerse the interventional instrument 90. Specifically, the volume of the ice water can be determined by the mark indication 1115 provided on the side wall of the liquid tank. It should be noted that the mark indication 1115 is set at a certain distance from the highest position on the top side of the liquid tank 111 to reduce the possibility of ice water overflowing due to incorrect operations.

A fixed mount 116 is provided in the liquid tank 111, and a matching structure is provided adjacent to the fixed mount 116 for detachable installation of an extension mount 117. The fixed mount 116 and the extension mount 117 both have positioning grooves 1113 that match the interventional instrument 90. The fixed mount 116 can be adapted to one or more types of interventional instruments 90, and the number of extension mounts 117 is selected according to different types of interventional instruments 90. Only the positioning grooves 1113 are different between the extension mounts 117. Therefore, one tray body 100 can be adapted to a plurality of models of interventional instruments, as long as the liquid tank 111 is large enough. Thus, only one large mold corresponding to the tray body 100 is required, which reduces the cost of die sinking compared with traditional packaging technologies.

In this embodiment, one tray body can be adapted to a plurality of extension mounts (corresponding to interventional instruments of different sizes), and the fixed mount of the tray body matched with the extension mounts can be adapted to one or more types of interventional instruments. Therefore, only one mold corresponding to the tray body is required, which reduces the cost of die sinking compared with traditional technologies. Moreover, there is no need to exert additional force during the preassembly and disassembly process of the delivering system, which at least avoids damage to the catheter component and the interventional instrument. The operator can load the interventional instrument while the delivering system is loaded in a blister box, which reduces the operator's error during loading and removal operations and improves efficiency.

The fixed mount 116 and the extension mount 117 can be made of the same or different materials as the tray body 100, such as PET (PETG, PETE) or PP.

In one embodiment, the matching structure includes:
a snapping block arranged on one of the extension mount and the bottom of the liquid tank; and
a snap-in groove provided on the other of the extension mount and the bottom of the liquid tank.

As shown in FIGS. 30 and 31, there are two groups of matching structures, which are arranged on two sides of the fixed mount 116. The clamping blocks 1112 are protruding from the bottom of the liquid tank 111, and the snap-in grooves 1111 are correspondingly provided in the extension mount 117.

Continuing to refer to FIGS. 30 and 31, according to an embodiment, the fixed mount 116 protrudes from the bottom of the liquid tank 111, the extension mount 117 covers the fixed mount 116, and at least parts of the fixed mount 116 and the extension mount 117 are complementary in shape. Complementary means that the outer periphery of the fixed mount 116 and the inner periphery of the extension mount 117 are engaged with and limited to each other, thereby improving the connection strength between the extension mount 117 and the tray body 100.

The fixed mount 116 includes two limiting protrusions 1119 protruding from the liquid tank 111. A positioning groove 1113 of the fixed mount 116 is formed between the two limiting protrusions 1119. The extension mount 117 has two limiting grooves 1118 complementary to the limiting protrusions 1119. In order to protect the valve from damage during packaging, the interventional instrument delivering system includes a crimping device 240 for accommodating the interventional instrument 90. As shown in FIGS. 9 and 10, the crimping device 240 has a cylindrical structure, and the positioning groove 1113 is matched with the crimping device 240 and has an arc-shaped inner wall.

The crimping device 240 includes a connection section 241 around the periphery of the catheter component 260, a transition section 242 for guiding the interventional instrument to be loaded into the catheter component, and an extension section 243 for receiving the interventional instrument in an expanded state, which are arranged from proximal to distal end. The extension section 243 covers the periphery of the interventional instrument 90 to prevent it from being exposed, thereby protecting the interventional instrument 90. The extension section 243 is provided with an opening for ice water to flow into and immerse the interventional instrument.

The catheter component 260 includes a guide head 261 extending distally from crimping device 240. The distal end of the guide head 261 is tapered to facilitate intervention in the human body. In order to facilitate the positioning and loading of the distal end of the catheter component 260, the distal end of the catheter component can be inserted through a support rod (such as the steel wire 262 in FIG. 32), including the steel wire 262 extending further distally from the guide head 261. The distal end of the steel wire 262 is provided with a positioning head 263. A positioning step 1116 is provided in the liquid tank 111. The positioning step 1116 has a limiting groove 1117 adapted to the positioning head 263. The spatial posture of the distal part of the catheter component can be maintained. The bottom of the liquid tank 111 is provided with a liquid drain port 1110, and the liquid drain port 1110 is provided with a plug 118. The operator can place the tray body 100 on the table, and after completing the loading of the interventional instrument 90, the plug 118 can be removed to drain the ice water 1114.

The bottom wall of the liquid tank 111 is flat or inclined, and the liquid drain port 1110 is preferably at the lowest point.

In order to ensure the positioning effect, a plurality of limiting members 120 are detachably installed on the top side 101 of the tray body 100. At least a part of the limiting members 120 spans over the accommodation area 110 to limit the interventional instrument delivering system 2 to the accommodation area 110 for easy transportation. The limiting member 120 has at least two ends connected to the tray body 100, and one or both ends can be operated during assembly and disassembly. The shape, size, etc. of the limiting members 120 are adapted to accommodation areas 110 of different shapes.

For traditional trays, especially the accommodation area which the catheter component is adapted to is provided with protrusions. During pre-assembly or disassembly of the catheter component, external force is required to deform the protrusions and insert the same into or out of the accommodation area, which may cause damage to the catheter component, so the operator needs to be careful to avoid damaging the catheter component, making the operation laborious. By contrast, in the present disclosure, the delivering system can be pre-assembled or removed easily, and the operator only needs to disassemble the limiting members before the removal operation. After removing the limiting members, there is no obstruction above the delivering system, so no damage would be caused to the delivering system. The operator does not need to worry about damaging the delivering system, and accordingly the operating efficiency can be improved.

The limiting member 120 is in a strip shape, and its two ends have engagement structures that cooperate with the top side 101 of the tray body 100. The difference between the plurality of limiting members 120 mainly lies in the length, in order to span different accommodation areas 110 (with different heights). At least the middle portion (located above the accommodation area 110) of the limiting member 120 is flexible and bendable.

The engagement structure includes a snap-in groove (not shown) at the end of the limiting member 120 or the top side 101 of the tray body 100, and a snapping post 122 that matches the snap-in groove. The snapping post 122 is correspondingly located on the top side 101 of the tray body 100 or the end of the limiting member 120. According to an embodiment, the snapping post 122 corresponding to at least one end of the limiting member 120 is cylindrical, so the limiting member 120 only needs to be disassembled at one end and then rotated to release the restriction on the delivering system 2.

Referring to FIG. 29, in this embodiment, the top side 101 of the tray body 100 has an installation surface 103 with a snapping post 122. The end of the limiting member 120 corresponding to the installation surface 103 is provided with a handle portion 123. The installation surface 103 is a horizontal surface, and the area is larger than one end of the limiting member 120 and the handle portion 123. The handle portion 123 is higher than the installation surface 103, making it easier for the operator to hold and remove the limiting member 120.

As mentioned above, the accommodation area 110 has different shapes to adapt to different positions of the delivering system 2. The specific accommodation area 110 includes a handle accommodation area 112, a catheter component accommodation area 113 and an interventional instrument accommodation area 114 serving as the liquid tank 111. The catheter component accommodation area 113 is a long and narrow groove. Compared with the catheter component accommodation area 113, the width of the liquid tank is increased and the bottom is deepened, which prevents ice water from flowing into other accommodation areas and ensures sufficient ice water.

A guide portion 1131 is provided at the interface between the catheter component accommodation area 113 and the liquid tank 111 to guide the catheter component 260 into the accommodation area. The guide portion 1131 has a V-shaped structure that opens upward (i.e., the top side is opened) to guide the catheter component 260 during pre-assembly. After part of the catheter component 260 is pre-assembled into the corresponding accommodation area, the rest of the catheter component 260 is pre-assembled accordingly.

Considering the volume of the tray body 100 and the arrangement between the catheter component 260 and the interventional instrument 90, the ice water may flow into other accommodation areas when the interventional instrument is immersed. According to an embodiment, in the catheter component accommodation area 113, a sealing plug 1132 is provided adjacent to the liquid tank 111 to prevent the flow of ice water. The catheter component accommodation area 113 has a corresponding sealing plug accommodation area 115 that is further enlarged outward (by deepening and/or widening). The sealing plug 1132 is shaped as a column (such as a cuboid or a cylinder) that is formed by one or more pieces and engages with the sealing plug accommodation area 115, so as to be easily inserted around the outer periphery of the catheter component.

In order to ensure the sealing effect, in a preferred embodiment, one of the limiting members 120 spans across and acts on the top of the sealing plug 1132, to prevent the sealing plug 1132 from loosening.

There are three groups of limiting members 120, corresponding to the handle accommodation area 112, the catheter component accommodation area 113, and the interventional instrument accommodation area serving as the liquid tank 111 respectively. As shown in FIG. 33, the first group of limiting member is located in the handle accommodation area 112, the second group of limiting member is located in the catheter component accommodation area 113, and the third group of limiting member is located in the liquid tank 111 and has at least one limiting member 120 spanning across the positioning groove 1113 and acting on the crimping device 240. The first group of limiting member includes at least two limiting members 120, respectively spanning across the proximal end and the distal end of the control handle 60.

The catheter component 260 includes a first section 2601 and a second section 2602 respectively connected to the control handle 60 and the interventional instrument 90, and a third section 2603 connected between the first section 2601 and the second section 2602. The first section 2601 and the second section 2602 are straight sections, and the third section 2603 is a curved section (the variation in the depth direction is not considered). Therefore, the length of the tray body 100 is not too long, and the length and width are appropriate, which facilitates packaging and transportation.

Therefore, the second group of limiting member includes at least three limiting members 120 respectively spanning across and acting on the first section, the second section and the third section. The catheter component accommodation area 113 is locally further recessed and extended outward to form an avoidance area 1191. The avoidance area 1191 allows a hand or tool to extend below the catheter component 260 and then lift the catheter component out of the accommodation area 110.

At least a part of the handle accommodation area 112 is further extended outward relative to the outer circumference of the control handle 60 to form an avoidance area 1121 relative to the control handle 60. In the traditional technologies, the control handle needs to be completely removed out for loading, while the other components are still in a pre-assembled state. The connection between the catheter component and the control handle is thus easy to be damaged, and the operator needs to be careful during the operation and thus the operation efficiency is low. By contrast, the avoidance area in this embodiment allows to operate the control handle for loading when the delivering system is pre-assembled or the control handle is partially removed. After loading is completed, the delivering system is removed.

As shown in FIGS. 38 and 39, the control handle 60 is connected to an extension tube 232 (for example, connected through a hose), which is used to connect the exhaust device 250 (such as a syringe or pump, etc.) for exhaust. In use, the proximal end of the control handle can be lifted and connected to the exhaust device 250 to complete the exhaust operation.

The catheter component 260 includes a catheter movably connected to the control handle 60, and the control handle 60 is provided with an operating portion 231 that can control the catheter. As shown in FIG. 32, there are a plurality of operating portions 231 which are only shown for example. To facilitate operation, the position of the avoidance area 1121 corresponds to the operating portions 231.

FIGS. 34 and 35 shows a tray according to any of the above embodiments, a sealing bag 104 for covering the tray, and a packaging box 105 containing the sealing bag 104 for transportation.

Among them, the packaging box 105 is provided with a temperature display 106, and a corresponding temperature sensor is configured inside for monitoring the temperature inside the box.

Especially when the heart valve prosthesis is stored with the valve dried, it is easier to monitor the storage environment. Similarly, humidity or other parameters can also be monitored.

FIGS. 12 to 14, or FIGS. 20 to 22 show operating the control handle 60 to load the interventional instrument. In view of this process, an embodiment of the present disclosure further discloses a loading method for the interventional instrument, which includes:
arranging the crimping device around the outside of the outer sheath, and releasing the distal end of the pulling wire to a sufficient length through the wire control tube; when the interventional instrument is in the expanded state, winding the engagement loop of the pulling wire around the retrieval cell of the stent, and then arranging the engagement loop around the corresponding lock member; driving the inner core so that the lock member engaged with the locking hole to prevent the pulling wire from falling off from the stent;
operating the control handle to drive the wire control tube to move proximally and tighten the pulling wire slightly, while the interventional instrument is still in an expanded state; proceeding packaging, storage and transportation;
during interventional surgery, opening the package; FIGS. 34 to 39 show the on-site loading process; before the operation, determining whether the packaging box 105 has experienced an unexpected temperature or time with the temperature display, as well as the integrity of the appearance of the packaging box 105; then opening the packaging box 105 and the sealing bag 104, and taking out the carrying tray 1; pouring ice water 1114 into the liquid tank 111 until the water level reaches the mark indication 1115, so as to completely immerse the interventional instrument;
moving the crimping device distally until the assembly indicator is basically aligned with the outer sheath orifice, switching the locking mechanism to the fastening position, where the interventional instrument is in an expanded state and located inside the crimping device, and has not entered the outer sheath yet;
using the control handle to drive the wire control tube and switching the pulling wire to the tightened state; the junction of the pulling wire and the interventional instrument is close to the lock seat, and the interventional instrument will be partially guided by the crimping device to be radially compressed; moving the sheath distally (the crimping device moves synchronously) so that the interventional instrument is gradually compressed by the crimping device from the proximal end to the distal end and is finally stored in the outer sheath; switching the locking mechanism to the release position and driving the crimping device to move distally to separate from the outer sheath, thereby completing loading; and
after completing loading, removing the plug 1111 to drain the ice water. The above loading method can also be regarded as the usage or operation method of the interventional instrument preloading and delivering system or the interventional instrument preloading and delivering assembly.

The interventional instrument preloading and delivering system in this disclosure can prevent the dry valve from being in a compressed state for a long time, and the overall system is easy to operate without the need for too much external tooling.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same figure, it can be considered that the figure also discloses a combined embodiment of various embodiments involved.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure.

## Claims

1. An interventional instrument preloading and delivering system, comprising:
a catheter component having opposing distal and proximal ends;
a crimping device having a cylindrical structure with an axis direction, wherein in the axis direction, one end of the crimping device surrounds a distal end of the catheter component, and the other end encloses a chamber with an inner conical surface;
an interventional instrument radially deformable and having relative compressed and expanded states;
a control handle connected to the proximal end of the catheter component; and
a pulling wire, one end of which is controlled by the control handle, and the other end of which extends distally in the catheter component and cooperates with the interventional instrument, wherein the pulling wire has a locked state and an unlocked state relative to the interventional instrument, in the locked state, the pulling wire is configured to pull the interventional instrument to transform from the expanded state to the compressed state, and in the unlocked state, the pulling wire is configured to be disengaged from and release the interventional instrument.

2. The interventional instrument preloading and delivering system according to claim 1, wherein the catheter component comprises:
an inner sheath, a distal end of which is provided with a locking portion;
an outer sheath slidably surrounding on an outside of the inner sheath, wherein the interventional instrument is configured to be loaded and received in the outer sheath in the compressed state; and
an inner core slidably arranged in the inner sheath, wherein one end of the inner core is an extension section extending out of the distal end of the inner sheath, a radial gap between the inner core and the inner sheath is a threading passage in which the pulling wire is movably arranged, and a lock member at a distal side of the locking portion is fixed on the extension section; the lock member has a locked position in which the lock member is engaged with the locking portion and an unlocked position in which the lock member is disengaged from the locking portion, and in the locked state, the pulling wire is wound around the interventional instrument and then around the lock member.

3. The interventional instrument preloading and delivering system according to claim 2, wherein a lock seat is fixed at the distal end of the inner sheath, and the locking portion is provided on the lock seat;
the extension section of the inner core is provided with a mounting seat, and a plurality of lock members are provided which are rod-shaped, each of which is connected to the mounting seat, and the lock members extend further proximally from the mounting seat; and
the lock seat or the mounting seat is further provided with a guide hole, and the pulling wire extends outward from the guide hole.

4. The interventional instrument preloading and delivering system according to claim 3, wherein the locking portion is a locking hole or a locking groove.

5. The interventional instrument preloading and delivering system according to claim 2, wherein the catheter component further comprises a wire control tube, the wire control tube is slidably fitted in the threading passage, and a proximal end of the pulling wire is fixedly connected to a distal end of the wire control tube and is controlled by the control handle through the wire control tube.

6. The interventional instrument preloading and delivering system according to claim 3, wherein relative to a distal port of the threading passage, when the lock member is in the locked position, the pulling wire has relative tightened and extended states;
when the pulling wire is in the tightened state, a joint between the interventional instrument and the pulling wire is close to the lock seat; and
when the pulling wire is in the extended state, the interventional instrument is in the expanded state and located in the crimping device, and the joint between the interventional instrument and the pulling wire is relatively far away from the lock seat.

7. The interventional instrument preloading and delivering system according to claim 3, wherein the lock member is surrounded by a slidable push block, a compression spring is arranged between the push block and the mounting seat, and an engagement portion between the lock member and the pulling wire is located at a proximal side of the push block.

8. The interventional instrument preloading and delivering system according to claim 7, wherein the push block is in a sheet shape and is provided with through holes for the lock members to pass through.

9. The interventional instrument preloading and delivering system according to claim 7, wherein a support sleeve is movably provided on the inner core, when the lock member is in the locked position, the support sleeve is in contact between the push block and the lock seat, and the lock member extends out of the push block to engage with the locking portion.

10. The interventional instrument preloading and delivering system according to claim 2, wherein a plurality of groups of pulling wires are provided, each group corresponds to a lock member and consists of a single pulling wire extending in one direction and having a wire loop at an end for movably surrounding the lock member; or
each group consists of a single wire extending back and forth, with a wire loop formed at a turning point for movably surrounding the lock member.

11. The interventional instrument preloading and delivering system according to claim 4, wherein the lock seat comprises a guide plate, a connecting sleeve and a fixed plate connected in sequence from a distal end to a proximal end, wherein the locking hole is opened in the fixed plate, the guide plate is provided with a guide hole corresponding to the locking hole; and
the lock member passes through the guide hole in the locked position, and is inserted into the corresponding locking hole after passing over a periphery of the connecting sleeve, wherein a section of the lock member corresponding to the periphery of the connecting sleeve serves as a working section, and the pulling wire is constrained to the working section.

12. The interventional instrument preloading and delivering system according to claim 2, wherein the crimping device has a cylindrical structure with an axis direction, in the axis direction, the cylindrical structure comprises:
a connection section having a length of 30 to 80 mm, one end of which is provided with a radially deformable locking mechanism;
a transition section enlarged relative to the connection section and having an inner conical surface for guiding the interventional instrument to radially deform, wherein the transition section is fixed to an end of the connection section away from the locking mechanism; and
an extension section, wherein the extension section and the transition section define a chamber for receiving the interventional instrument in the expanded state.

13. The interventional instrument preloading and delivering system according to claim 12, wherein a distal end of the outer sheath has a diameter-enlarged section for accommodating the interventional instrument, and the locking mechanism acts on a proximal end of the diameter-enlarged section.

14. The interventional instrument preloading and delivering system according to claim 12, wherein the locking mechanism comprises:
a plurality of elastic claws located at an end of the connection section and distributed in a circumferential direction of the connection section at intervals; and
a sliding sleeve surrounding an outer periphery of the plurality of elastic claws and slidably matched with the connection section, wherein the sliding sleeve has relative fastening and release positions on its own sliding path, and wherein in the fastening position, the sliding sleeve holds and exerts force on the plurality of elastic claws inward, and in the release position, the sliding sleeve releases the force on the plurality of elastic claws.

15. The interventional instrument preloading and delivering system according to claim 12, wherein the locking mechanism comprises:
an elastic ring located inside the connection section; and
a lock cap threadedly engaged with the connection section, wherein at least a part of the lock cap extends inside the connection section and abuts the elastic ring.

16. The interventional instrument preloading and delivering system according to claim 12, wherein in the axis direction, the chamber has a length greater than or equal to that of the interventional instrument in the expanded state.

17. The interventional instrument preloading and delivering system according to claim 12, wherein the transition section and the extension section are formed in separate pieces, and a junction of the two is located at a position of the chamber adjacent to the connection section.

18. The interventional instrument preloading and delivering system according to claim 5, wherein the outer sheath, inner sheath, wire control tube and inner core respectively extend to the control handle, wherein:
a proximal end of the inner sheath is fixedly connected to the control handle; and
proximal ends of the outer sheath, the wire control tube and the inner core are movably connected to the control handle and are axially slidable relative to the control handle, and three driving mechanisms are provided in the control handle that are respectively in transmission fit with the outer sheath, the wire control tube and the inner core.

19. The interventional instrument preloading and delivering system according to claim 1, wherein the interventional instrument is an optional heart valve prosthesis and includes a stent and leaflets, the stent generally has a meshed cylindrical structure with a blood flow channel therein, and the leaflets cooperate with each other in the blood flow channel to open or close the blood flow channel; each leaflet comprises a fixed edge fixed to the stent, and a free edge for cooperating with other leaflets to control the blood flow channel;
the stent comprises:
an annular part, the annular part has a radially deformable structure, two ends of the annular part in its own axial direction are a first end and a second end respectively, wherein an edge of the first end comprises a plurality of cell sections arranged in sequence in a circumferential direction of the annular part; and
a plurality of guide parts arranged at intervals in the circumferential direction of the annular part, one side of each guide part is connected to a corresponding cell section, and the other side of each guide part gradually tapers to an end, at which end a retrieval cell is provided for the pulling wire to pass through.

20. The interventional instrument preloading and delivering system according to claim 19, wherein the first end of the annular part is formed by connected ribs with peaks and valleys; each cell section comprises at least 3 to 7 peaks; and
the peaks are directly connected to the corresponding guide parts.

21. The interventional instrument preloading and delivering system according to claim 20, wherein in an outflow direction, the guide part comprises a second region and a first region;
the first region is the retrieval cell for the pulling wire to pass through, and
the second region comprises a second cell for fixing ears of the leaflets.

22. The interventional instrument preloading and delivering system according to claim 21, wherein the second cell has a top end and a bottom end, the top end is connected to a bottom end of the retrieval cell, and the bottom end is connected to the peak of the corresponding cell section positioned centrally in the circumferential direction of the corresponding cell section.

23. The interventional instrument preloading and delivering system according to claim 19, wherein the guide parts are evenly arranged in the circumferential direction of the annular part; both the guide parts and the annular part have hollowed cell structures; in the axial direction of the annular part, the annular part comprises a plurality of rows of cells; and an interface between the guide part and the annular part is formed by vertices of one circle of cells; and
depending on the blood flow direction controlled by the leaflets, the first end is an outflow end, and the second end is an inflow end, wherein two ends of the fixed edge are respectively located on two adjacent guide parts, and a middle portion of the fixed edge extends to the annular part.

24. The interventional instrument preloading and delivering system according to claim 19, wherein both the guide parts and the annular part have hollowed cell structures, and cells of each guide part are divided into relative sparse and dense areas; and at least a part of the sparse area is close to an opening between two adjacent guide parts.

25. The interventional instrument preloading and delivering system according to claim 24, wherein the guide part includes four closed regions, each closed region corresponds to a cell, and the four closed regions are respectively:
a first region being the retrieval cell;
a second region, in the circumferential direction of the annular part, the second region is aligned with the first region and serves as a central region of the guide part;
a third region located on one side of the central region in the circumferential direction of the annular part; and
a fourth region located on the other side of the central region in the circumference of the annular part.

26. The interventional instrument preloading and delivering system according to claim 24, wherein,
a second cell is provided in the second region, and the second cell is used to fix ears of the leaflets.

27. The interventional instrument preloading and delivering system according to claim 26, wherein the cell in the third and fourth regions adjacent to the first and second regions is a third cell, and the third cell has the largest area among all cells of the guide part.

28. The interventional instrument preloading and delivering system according to claim 19, wherein an inner side of the stent is provided with an inner covering film, and the inner covering film is located on an inflow end of the leaflets and is fixed with the fixed edges of the leaflets; and
a perivalvular leakage preventing component is fixed on an outside of the inner covering film, and the perivalvular leakage preventing component consists of spaced pieces corresponding to the cells.

29. The interventional instrument preloading and delivering system according to claim 28, wherein the perivalvular leakage preventing component and the inner covering film are formed in one piece, and in the expanded state, the perivalvular leakage preventing component extends outward in a radial direction of the stent from the corresponding cells.

30. The interventional instrument preloading and delivering system according to claim 28, wherein the inner covering film is made of PET material, and the perivalvular leakage preventing component is made of porous material.

31. The interventional instrument preloading and delivering system according to claim 28, wherein the perivalvular leakage preventing component is located on an inflow end of the stent, different portions of the same piece of perivalvular leakage preventing component have different protruding heights, and the highest protruding portion is closer to an inflow end of the corresponding cell.

32. The interventional instrument preloading and delivering system according to claim 31, wherein a distance between the highest protruding portion of the same piece of perivalvular leakage preventing component and the inflow end of the corresponding cell is S1, and a distance between the highest protruding portion of the same piece of perivalvular leakage preventing component and an outflow end of the corresponding cell is S2, wherein S1 : S2 is 0 to 0.8.

33. The interventional instrument preloading and delivering system according to claim 28, wherein the same piece of perivalvular leakage preventing component gradually becomes thicker from the outflow end toward the inflow end, and then gradually becomes thinner after reaching the highest protruding portion.

34. An interventional instrument preloading and delivering system, comprising:
a catheter component having opposing distal and proximal ends;
a crimping device having a cylindrical structure with an axis direction, wherein in the axis direction, one end of the crimping device surrounds a distal end of the catheter component, and the other end encloses a chamber with an inner conical surface;
an interventional instrument comprising a stent with a cylindrical structure and having relative compressed and expanded states, the interventional instrument is configured to be located in the chamber in the expanded state, the stent comprising:
an annular part, the annular part has a radially deformable structure, two ends of the annular part in its own axial direction are a first end and a second end respectively, wherein an edge of the first end comprises a plurality of cell sections arranged in sequence in a circumferential direction of the annular part; and
a plurality of guide parts arranged at intervals in the circumferential direction of the annular part, one side of each guide part is connected to a corresponding cell section, and the other side of each guide part gradually tapers to an end, at which end a retrieval cell is provided;
a control handle connected to the proximal end of the catheter component; and
a pulling wire, one end of which is controlled by the control handle and the other end extends distally in the catheter component and engages with the retrieval cell of the interventional instrument; relative to the interventional instrument, the pulling wire has a locked state and an unlocked state, in the locked state, the pulling wire is configured to pull the interventional instrument to transform from the expanded state to the compressed state, and in the unlocked state, the pulling wire is configured to be disengaged from and release the interventional instrument.

35. An interventional instrument preloading and delivering assembly, comprising:
a packaging component at least comprising a carrying tray, the carrying tray comprising a tray body, the tray body having opposing top and bottom sides, and the top side of the tray body having a recessed portion that forms an accommodation area; and
an interventional instrument preloading and delivering system, the interventional instrument preloading and delivering system is the interventional instrument preloading and delivering system according to any one of claims 1 to 34, and is located in the accommodation area.

36. The interventional instrument preloading and delivering assembly according to claim 35, wherein the accommodation area comprises:
a handle accommodation area, the control handle is located in the handle accommodation area;
an interventional instrument accommodation area, the crimping device and the interventional instrument are located in the interventional instrument accommodation area; and
a catheter component accommodation area, at least a part of the catheter component is located in the catheter component accommodation area.

37. A loading method for an interventional instrument, comprising:
maintaining the interventional instrument in an expanded state and connecting it to a control handle through a pulling wire;
providing a crimping device with an inner conical surface around an outer periphery of the interventional instrument, pulling the pulling wire, and gradually compressing the interventional instrument radially under guidance of the inner conical surface; and
receiving the radially compressed portion of the interventional instrument into an outer sheath until the interventional instrument completely enters the outer sheath.
